# EUROPEAN PATENT APPLICATION

(11) **EP 4 778 915 A1**
(43) Date of publication of application: **22.07.2026**
(21) Application number: 24864652.3
(22) Date of filing: 11.09.2024
(51) Int. Cl.: C07C 229/16, C07C 219/06, C07C 235/10, A61K 9/127, A61K 47/16, A61K 47/14

(54) **MATERIAL FOR LOCAL DRUG DELIVERY, AND USE THEREOF**

(30) Priority: 11.09.2023 CN 202311166483
(71) Applicant: DSCILAB.CO., LTD, Suzhou, Jiangsu 215000 (CN)
(72) Inventor: CUI, Zhiping, Suzhou, Jiangsu 215000 (CN); LIU, Xiaohua, Suzhou, Jiangsu 215000 (CN); WANG, Songli, Suzhou, Jiangsu 215000 (CN); CHEN, Yuanyuan, Suzhou, Jiangsu 215000 (CN); SUN, Sijie, Suzhou, Jiangsu 215000 (CN)
(74) Representative: Ipsilon
(86) International application number: PCT/CN2024/118226
(87) International publication number: WO 2025/055936

(57) **Abstract**

The present invention relates to a material for local drug and use thereof. Specifically provided is a nanoparticle composition comprising a lipid component, the lipid component comprising arepresented by formula (I). The nanoparticle composition of the present invention is delivered only at the site of administration.

## Description

### CROSS-REFERENCE TO RELATED APPLICATIONS

This application claims priority to Chinese Patent Application No. CN202311166483.8, filed on September 11, 2023, entitled "Materials for Local Drug Delivery and Uses Thereof," the entire disclosure of which is incorporated herein by reference.

### TECHNICAL FIELD

The present disclosure relates to the field of biomedical technology, specifically to materials for local drug delivery and uses thereof.

### BACKGROUND

The success of COVID-19 mRNA vaccines has revealed the immense potential of mRNA therapies delivered via lipid nanoparticles (LNPs). Currently marketed LNPs (BioNTech's BNT162b2 and Moderna's mRNA-1273) lead to significant expression of target proteins in human livers following various routes of administration (intravenous, subcutaneous, intramuscular injection). While these systems can play an important role in treating liver diseases, for extrahepatic lesions, these delivery systems carry potential toxic side effects, which are detrimental to clinical translation and application. There remains a need in the art for specific LNP delivery carriers with low toxicity.

### SUMMARY

A first aspect of the present disclosure provides a compound of Formula (I), 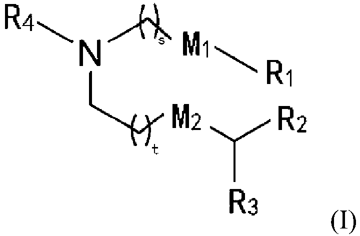 or a salt or isomer thereof, wherein:
s is 5 or 8, t is 7 or 8;
M₁ is -C(O)O- or -C(O)NR-;
M₂ is -C(O)O- or -C(O)NR₅-;
R₁ is C₁₋₁₈ alkyl, C₂₋₁₈ alkenyl, or -CHR₂R₃;
R₂, R₃, and R₅ are each independently selected from: H, C₁₋₁₄ alkyl, and C₂₋₁₄ alkenyl;
R₄ is C₁₋₃ alkyl, -(CH₂)ₙCHR₆R₇, or -CHR₆R₇, wherein R₆ and R₇ are each independently (CH₂)ₙQ, wherein n is 0, 1, 2, 3, or 4, Q is C₁₋₃ alkyl or OR, and R is C₁₋₃ alkyl or H.

Another aspect of the present disclosure provides a compound of Formula (Ia), 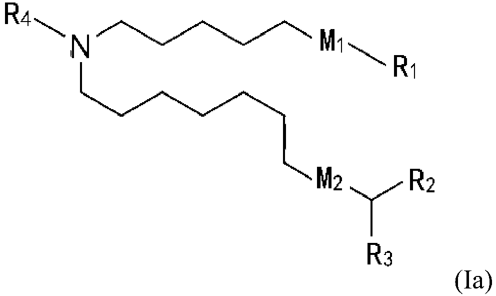 or a salt or isomer thereof, wherein:
s is 5 or 8, t is 7 or 8;
M₁ is -C(O)O- or -C(O)NR-;
M₂ is -C(O)O- or -C(O)NR₅-;
R₁ is C₁₋₁₈ alkyl, C₂₋₁₈ alkenyl, or -CHR₂R₃;
R₂, R₃, and R₅ are each independently selected from: H, C₁₋₁₄ alkyl, and C₂₋₁₄ alkenyl;
R₄ is C₁₋₃ alkyl, -(CH₂)ₙCHR₆R₇, or -CHR₆R₇, wherein R₆ and R₇ are each independently (CH₂)ₙQ, wherein n is 0, 1, 2, 3, or 4, Q is C₁₋₃ alkyl or OR, and R is C₁₋₃ alkyl or H.

In one or more embodiments, M₁ is -C(O)O- or -C(O)NH-.

In one or more embodiments, M₂ is -C(O)NR₅-, R₂ is H, R₃ is C₆ alkyl, and R₅ is C₇ alkyl.

In one or more embodiments, R₁ is C₁₋₁₄ alkyl.

In one or more embodiments, Q is OH.

In one or more embodiments, R₂ and R₃ are each independently selected from C₁₋₁₂ alkyl.

The present disclosure further provides a nanoparticle composition comprising a lipid component, wherein the lipid component comprises a compound according to any embodiment described herein (e.g., a compound of Formula (I)).

In one or more embodiments, the lipid component further comprises a phospholipid (e.g., a polyunsaturated lipid), a PEG lipid, and a structural lipid.

In one or more embodiments, the nanoparticle composition further comprises a therapeutic and/or prophylactic agent.

The present disclosure further provides a pharmaceutical composition comprising the nanoparticle composition according to any embodiment herein and a pharmaceutically acceptable excipient. The pharmaceutical composition is a locally acting pharmaceutical composition.

The present disclosure further provides a method of delivering a therapeutic and/or prophylactic agent to a cell, wherein the method comprises administering (e.g., injecting) to a subject a nanoparticle composition comprising: (1) a lipid component comprising a phospholipid (e.g., a polyunsaturated lipid), a PEG lipid, a structural lipid, and a compound of Formula (I), and (2) a therapeutic and/or prophylactic agent, wherein the administering comprises contacting the cell with the nanoparticle composition, thereby delivering the therapeutic and/or prophylactic agent to the cell.

In one or more embodiments, the therapeutic and/or prophylactic agent is a polynucleotide.

In one or more embodiments, the therapeutic and/or prophylactic agent is an mRNA or siRNA.

In one or more embodiments, the cell is a mammalian cell.

In one or more embodiments, the delivery is local (rather than systemic) delivery.

In one or more embodiments, the cell is proximal to the administration site.

In one or more embodiments, the cell is a cell of an organ or tissue at the administration site. The organ or tissue is, for example, a liver, spleen, lung, muscle, or femur.

In one or more embodiments, the subject is a mammal, such as a human.

The present disclosure also provides a method for producing a polypeptide in a cell, wherein the method comprises contacting the cell with a nanoparticle composition comprising: (1) a lipid component comprising a phospholipid (e.g., a polyunsaturated lipid), a PEG lipid, a structural lipid, and a compound of Formula (I), or a salt or isomer thereof, according to any embodiment herein, and (2) an mRNA encoding the polypeptide, whereby the mRNA is capable of being translated in the cell to produce the polypeptide.

In one or more embodiments, the cell is a mammalian cell.

The present disclosure further provides a method for treating a disease or disorder in a mammal (e.g., a human), or of enabling a therapeutic and/or prophylactic agent to act locally, by local (rather than systemic) administration of the therapeutic and/or prophylactic agent. The method comprises local administration (e.g., injection) of a therapeutically effective amount of a nanoparticle composition to the mammal. The nanoparticle composition comprises: (1) a lipid component comprising a phospholipid (e.g., a polyunsaturated lipid), a PEG lipid, a structural lipid, and a compound of Formula (I), or a salt or isomer thereof, according to any embodiment herein, and (2) a therapeutic and/or prophylactic agent (e.g., an mRNA).

In one or more embodiments, the disease or disorder is associated with dysfunction or abnormality of a protein or polypeptide.

In one or more embodiments, the disease or disorder is selected from rare diseases, infectious diseases, cancers and proliferative diseases, genetic diseases (e.g., cystic fibrosis), autoimmune diseases, diabetes, neurodegenerative diseases, cardiovascular diseases, renovascular diseases, and metabolic diseases.

The present disclosure also provides a method of local (rather than systemic) delivery of the therapeutic and/or prophylactic agent to a mammal. The method comprises administering a nanoparticle composition to a subject (e.g., a mammal), wherein the nanoparticle composition comprises: (1) a lipid component comprising a phospholipid, a PEG lipid, a structural lipid, and a compound of Formula (I), or a salt or isomer thereof, according to any embodiment herein, and (2) a therapeutic and/or prophylactic agent (e.g., an mRNA), wherein the administering step comprises contacting a mammalian cell with the nanoparticle composition, thereby delivering the therapeutic and/or prophylactic agent to a local region of the mammal.

In one or more embodiments, the local region is a target organ or tissue, such as a liver, spleen, lung, muscle, or femur.

The present disclosure also provides a method of preparing the compound of Formula (I) as described herein.

The present disclosure also provides a method of preparing the nanoparticle composition as described herein. The method comprises mixing (e.g., via microfluidization) the components of the nanoparticle composition.

In one or more embodiments, the method comprises mixing (e.g., via microfluidization) the components of the nanoparticle composition as described herein (e.g., a lipid of Formula (I), a phospholipid, a PEG lipid, a structural lipid, and an RNA), wherein the weight ratio of the lipid component to the therapeutic and/or prophylactic agent is from about 5:1 to about 60:1.

The present disclosure also provides use of the compound of Formula (I), or a salt or isomer thereof, according to any embodiment herein, or the nanoparticle composition according to any embodiment herein, in the manufacture of a locally acting drug for treating or preventing a disease or disorder.

In one or more embodiments, the disease or disorder benefits from local (rather than systemic) delivery of the therapeutic and/or prophylactic agent.

In one or more embodiments, the drug is for local (rather than systemic) delivery of a therapeutic and/or prophylactic agent.

In one or more embodiments, the nanoparticle composition, or a nanoparticle composition formed from the compound of Formula (I), is for delivering a therapeutic and/or prophylactic agent to a local (rather than systemic) region.

In one or more embodiments, the local region is a target organ or tissue, such as a liver, spleen, lung, muscle, or femur.

### BRIEF DESCRIPTION OF THE DRAWINGS

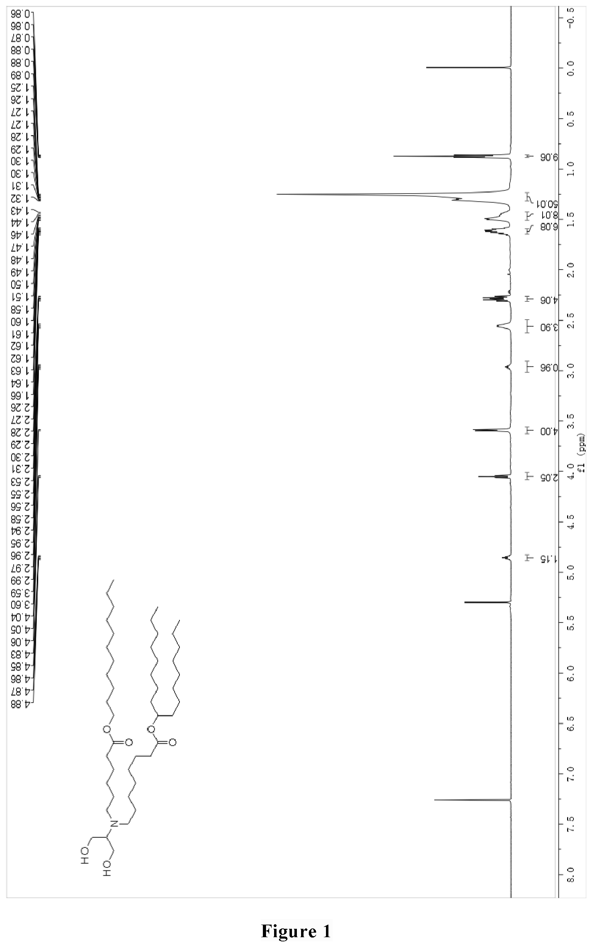

**Figure 1** shows the ¹H NMR spectrum of Compound 1.

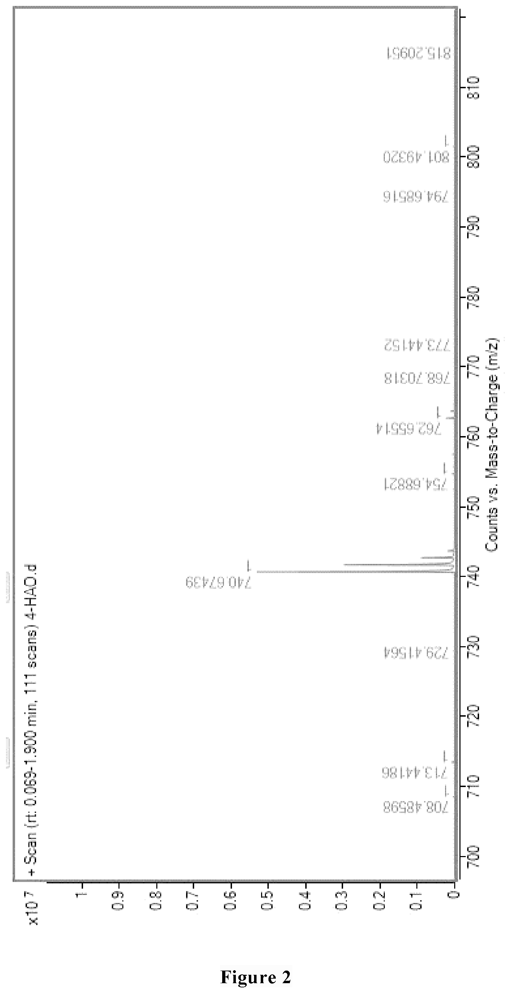

**Figure 2** shows the mass spectrum of Compound 1.

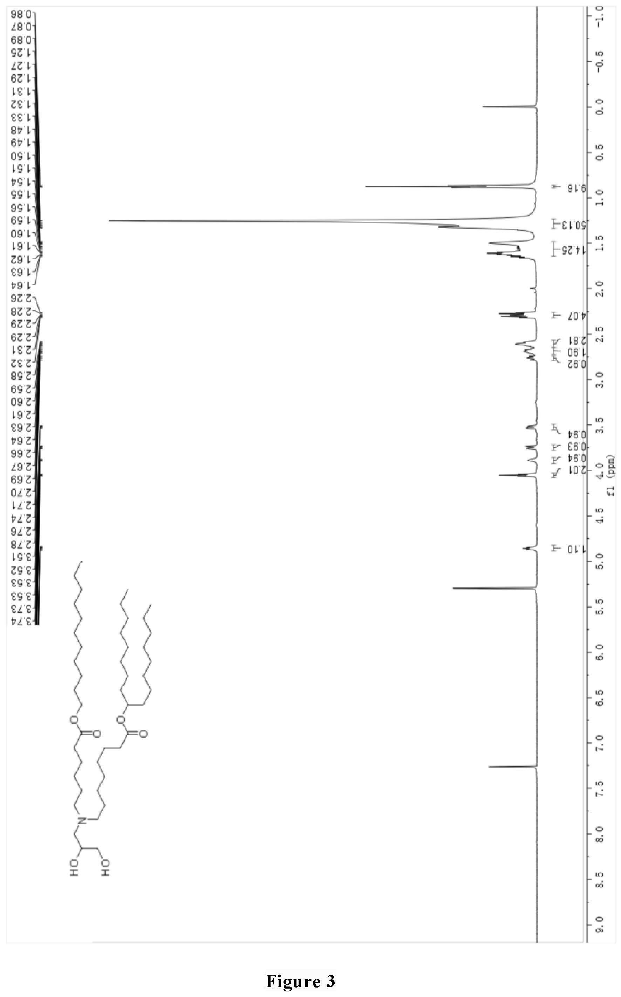

**Figure 3** shows the ¹H NMR spectrum of Compound 2.

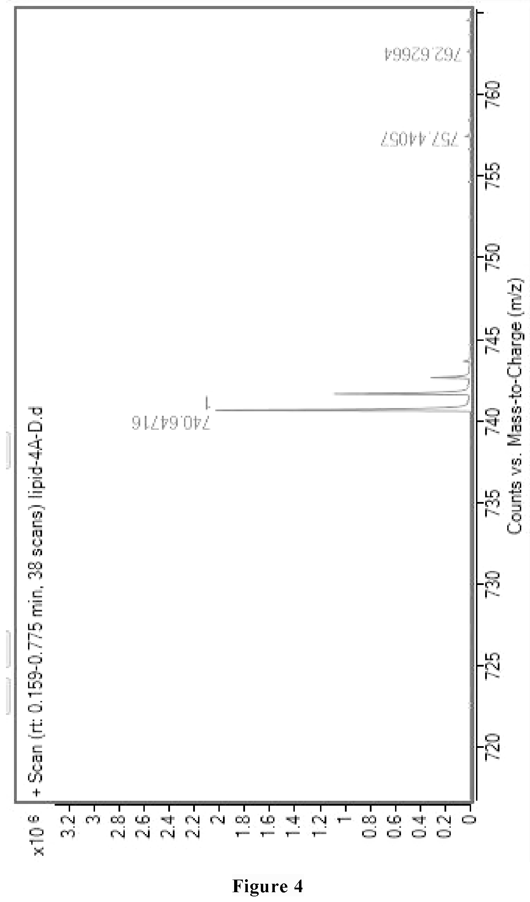

**Figure 4** shows the mass spectrum of Compound 2.

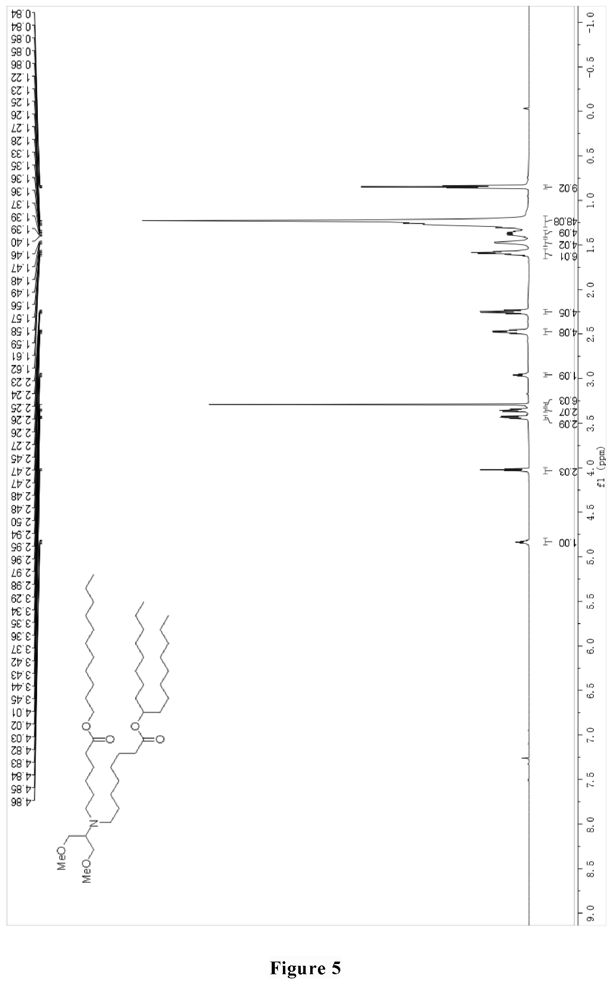

**Figure 5** shows the ¹H NMR spectrum of Compound 3.

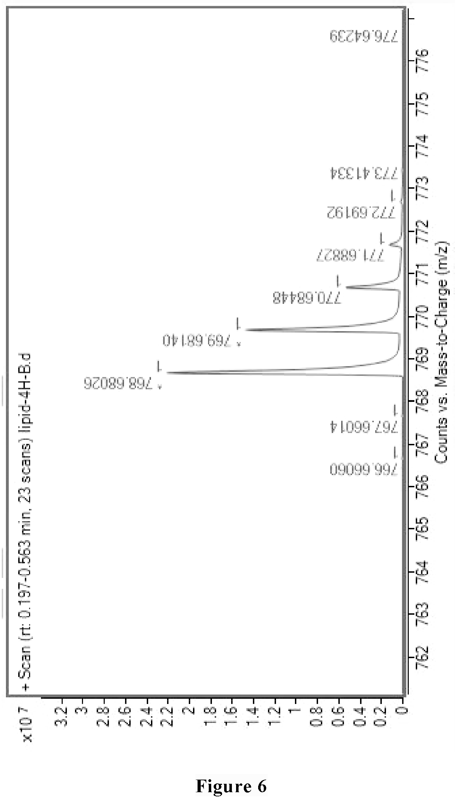

**Figure 6** shows the mass spectrum of Compound 3.

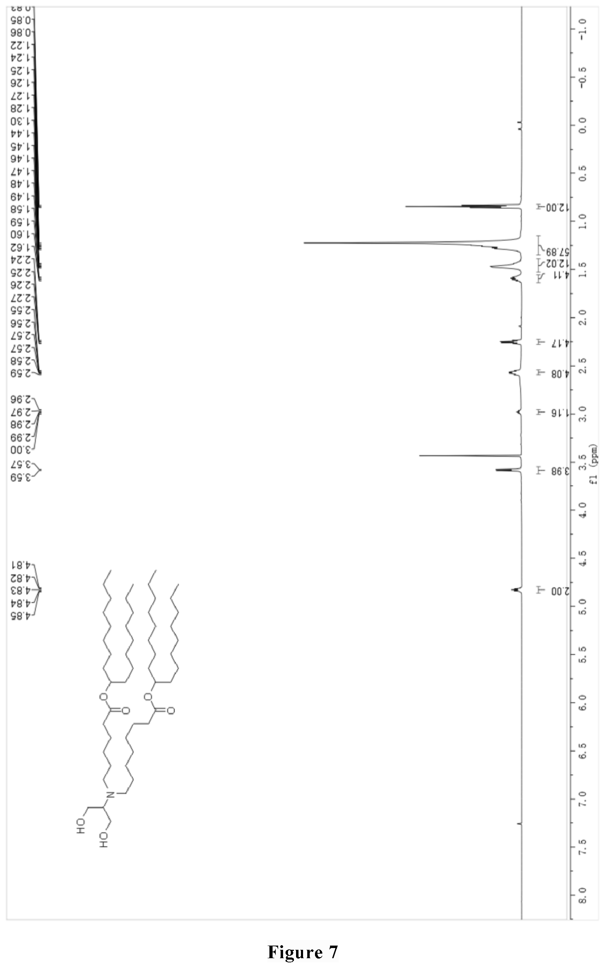

**Figure 7** shows the ¹H NMR spectrum of Compound 4.

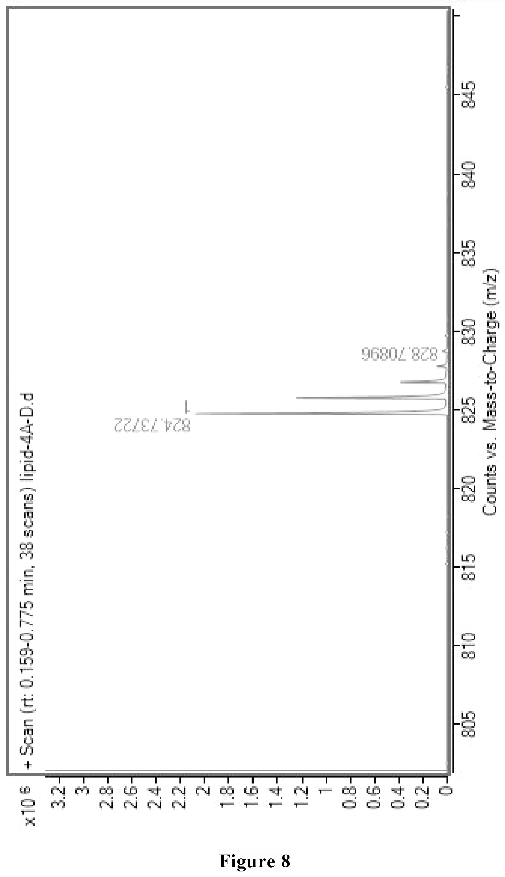

**Figure 8** shows the mass spectrum of Compound 4.

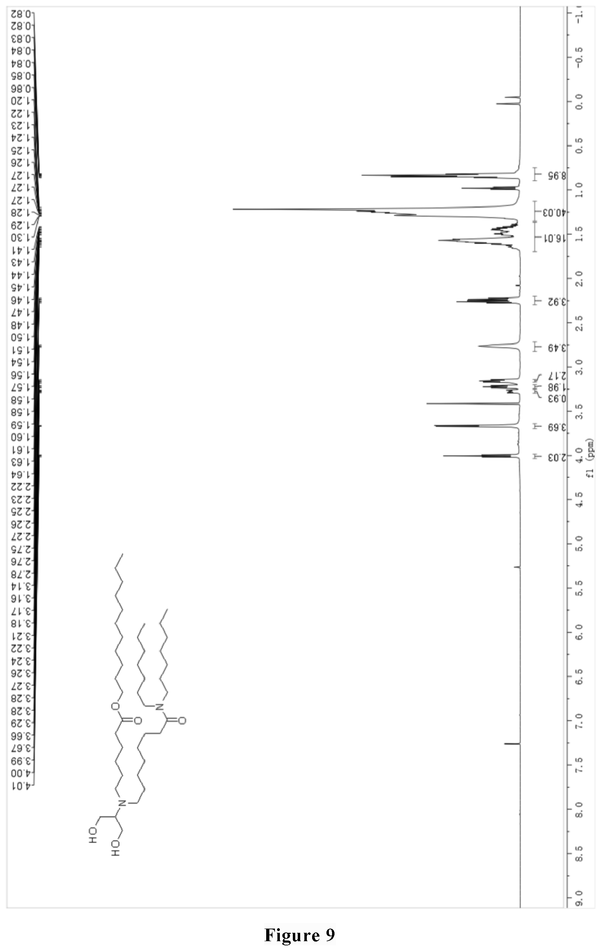

**Figure 9** shows the ¹H NMR spectrum of Compound 5.

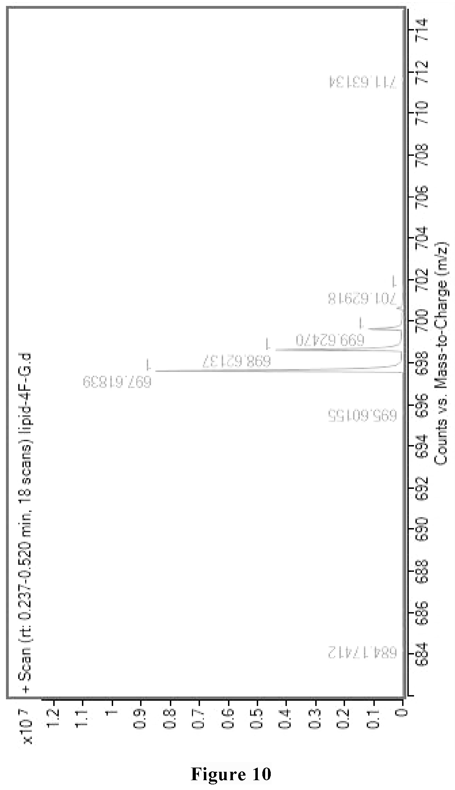

**Figure 10** shows the mass spectrum of Compound 5.

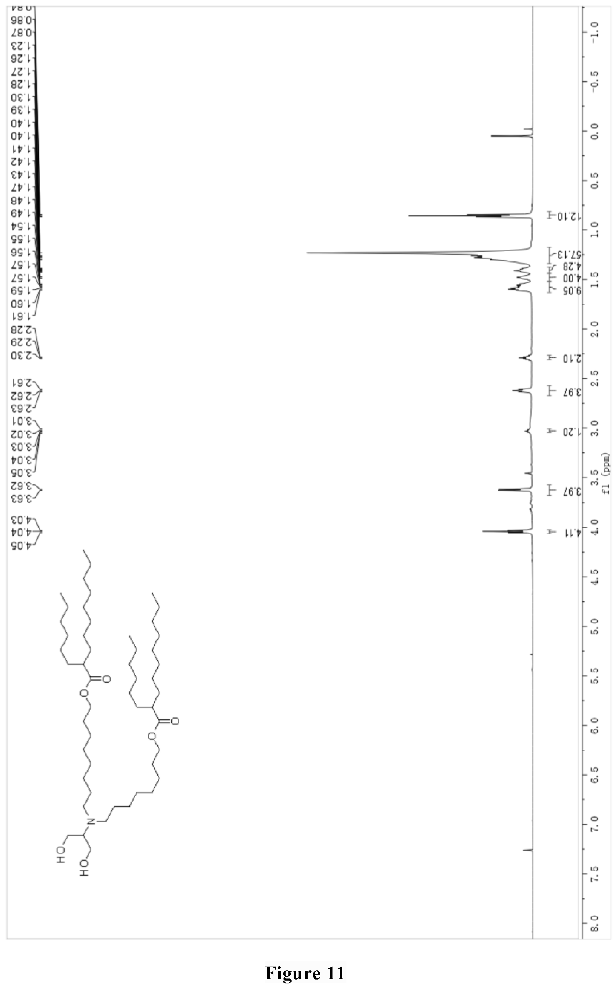

**Figure 11** shows the ¹H NMR spectrum of Compound 6.

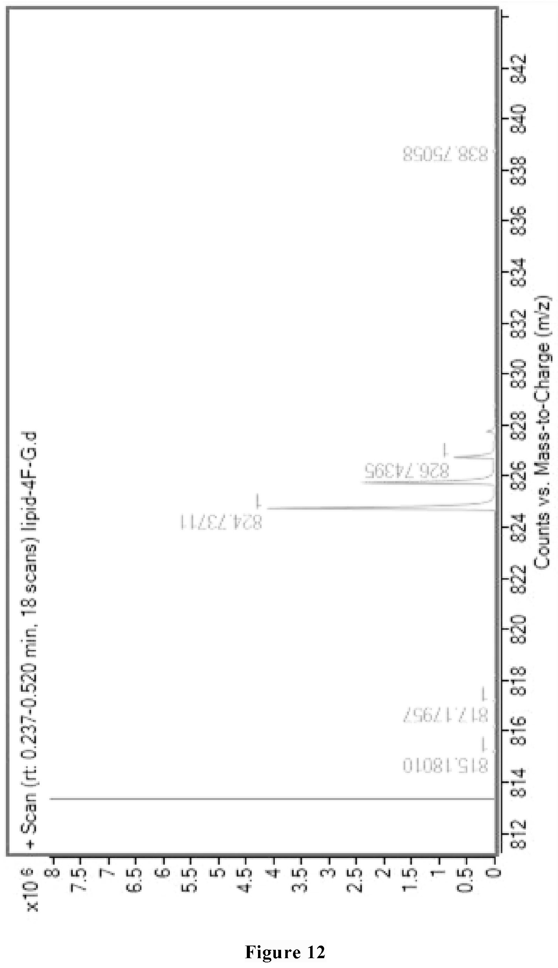

**Figure 12** shows the mass spectrum of Compound 6.

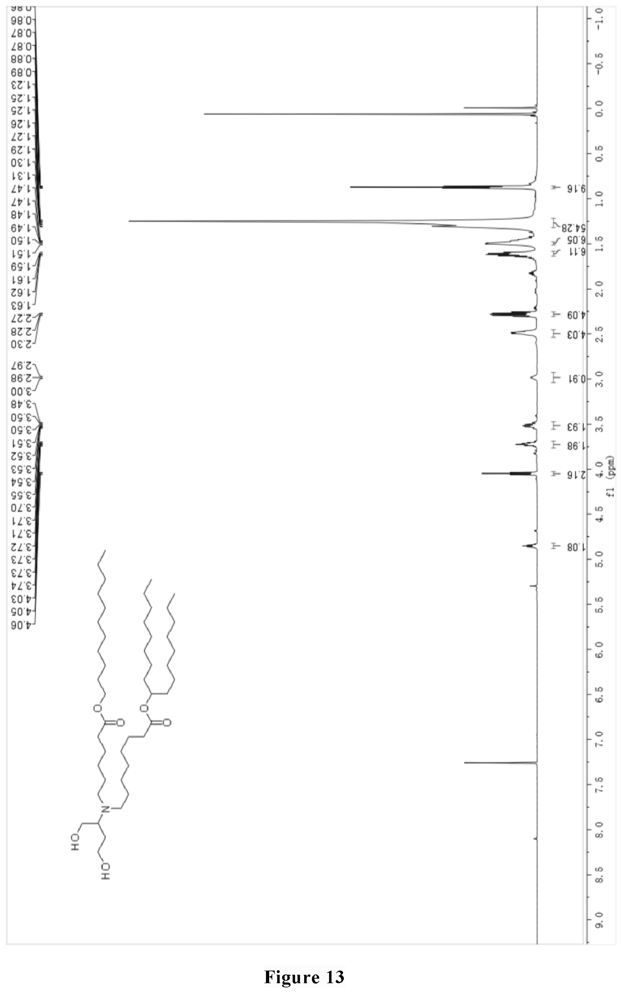

**Figure 13** shows the ¹H NMR spectrum of Compound 7.
**Figure 14** shows the mass spectrum of Compound 7.
**Figure 15** shows the particle size distribution of Compound 1 LNP. The particle size of Compound 1 LNP is 66.47 ± 1.368 nm, with a PDI of 0.084 ± 0.045.
**Figure 16** shows the particle size distribution of SM-102 LNP. The particle size of SM-102 LNP is 70.38 ± 0.468 nm, with a PDI of 0.055 ± 0.011.
**Figure 17** shows in vivo and ex vivo fluorescence semi-quantitative data after intramuscular injection. High fluorescence intensity was observed at the intramuscular injection site and the liver for SM-102 LNP, whereas for Compound 1 LNP, high fluorescence intensity was observed only at the intramuscular injection site, with no detectable fluorescence in the liver, indicating the superior local expression performance of Compound 1 LNP.
**Figure 18** shows the expression results in C57 mice after intramuscular injection of Compound 1 LNP. Significant expression was observed only at the intramuscular injection site for Compound 1 LNP, with no expression detected in the liver, indicating the superior local expression performance of Compound 1 LNP.
**Figure 19** shows the change in expression activity over time and the local expression area after intramuscular injection of Compound 1 LNP in C57 mice. The expression area of Compound 1 LNP decreased over time post-intramuscular injection, and throughout the experimental period, the expression site was confined solely to the injection site.
**Figure 20** shows the change in expression activity over time and the local expression area after intramuscular injection of Compound 1 LNP in SD rats. The expression area of Compound 1 LNP decreased over time post-intramuscular injection, and throughout the experimental period, the expression site was confined solely to the injection site.
**Figure 21** shows the expression results in C57 mice after intramuscular injection of SM-102 LNP. Expression was observed at both the intramuscular injection site and in the liver for SM-102 LNP, indicating that SM-102 LNP lacks local expression capability.
**Figure 22** shows the expression results after intrasplenic injection of Compound 1 LNP in C57 mice. Significant expression was observed only at the intrasplenic injection site for Compound 1 LNP, with no expression observed in other sites, indicating the superior local expression capability of Compound 1 LNP.
**Figure 23** shows the expression results after intrakidney injection of Compound 1 LNP in C57 mice. Significant expression was observed only at the intrakidney injection site for Compound 1 LNP, with no expression observed in other sites, indicating the superior local expression capability of Compound 1 LNP.
**Figure 24** shows the expression results after intrasplenic injection of Compound 1 LNP in SD rats. Significant expression was observed only at the intrasplenic injection site for Compound 1 LNP, with no expression observed in other sites, indicating the superior local expression capability of Compound 1 LNP.
**Figure 25** shows the expression results after intrakidney injection of Compound 1 LNP in SD rats. Significant expression was observed only at the intrakidney injection site for Compound 1 LNP, with no expression observed in other sites, indicating the superior local expression capability of Compound 1 LNP.

### DETAILED DESCRIPTION

The present disclosure synthesizes a novel ionizable cationic lipid and utilizes the novel lipid to prepare a lipid nanoparticle (LNP) delivery system. After encapsulation of Luciferase mRNA, this novel delivery system was injected into the mouse leg muscle, spleen, and kidney. In vivo fluorescence imaging in small animals showed that this delivery system enables protein expression of the encapsulated mRNA exclusively at the injection site, with no expression observed in other major organs (the heart, liver, spleen, lung, kidneys). The LNP delivery system provided by the present disclosure can overcome the defect of existing LNPs having strong liver tropism, providing an ideal delivery vehicle for achieving local specific expression via mRNA therapy. The organ-specific LNP delivery vehicle of the present disclosure provides a novel development platform for treating currently refractory or untreatable diseases, greatly expanding the clinical application of mRNA therapy.

The present disclosure relates to novel lipids and lipid nanoparticle compositions comprising novel lipids. The present disclosure also provides methods of delivering a therapeutic and/or prophylactic agent to mammalian cells, particularly delivering a therapeutic and/or prophylactic agent to a local region of a mammal, producing a target polypeptide in local cells of a mammal, and treating a disease or disorder in a mammal.

The present disclosure relates to a method of delivering a therapeutic and/or prophylactic agent to mammalian cells, wherein the method comprises administering to a subject a nanoparticle composition comprising the therapeutic and/or prophylactic agent, wherein the administering involves contacting cells or local regions with the composition, thereby delivering the therapeutic and/or prophylactic agent to the cells or the local regions.

As used herein, the term "local region" refers to cell(s), tissue(s), or organ(s) within a certain range of the administration site. In the prior art, special dosage forms are typically required to retain drugs at the application site, such as creams, ointments, sprays, and aerosols, mostly for external or mucosal surfaces. However, the inventors have found that medicaments (e.g., the nanoparticle composition or pharmaceutical composition described herein) prepared using the compound of Formula (I) described herein can be locally applied and locally acting in organs, tissues, or cells. In some embodiments, the nanoparticle composition of the present disclosure is suitable for preparing locally applied, locally acting products (LALAP). As used herein, the term "locally acting medicament(s)", "locally acting pharmaceutical composition(s)", "locally applied, locally acting product(s)", "local administration and local action", or "locally applied, locally acting" refers to a medicament applied locally that exerts its effect at the local region of application. Systemic effects (e.g., liver tropism) of such medicaments are considered unintended medicament actions. The local region can include only cells, only an organ (e.g., a liver, spleen, kidney, or lung), or only a tissue (e.g., a muscle, skin, or bone). In some embodiments herein, "local region" refers to only the cells, tissues, or organs at the administration site. In some embodiments, when local administration is performed to an organ (e.g., a liver, spleen, kidney, or lung), the medicament is delivered only within that organ at the administration site; when local administration is performed to a tissue (e.g., a specific muscle), the medicament is delivered only within that tissue (e.g., that muscle) at the administration site; when local administration is performed to cells, the medicament is delivered only to the cells within the organ or tissue at the administration site (e.g., a muscle where the cells reside). In some embodiments, when administered to a gastrocnemius muscle, the medicament is delivered only within the gastrocnemius muscle.

In some embodiments, the term "local region" or "locally" can mean that within 4-8 hours (e.g., 6 hours) after administration of the lipid nanoparticle (LNP) composition described herein to an organ (e.g., a liver, spleen, kidney, or lung), the LNP composition, the therapeutic and/or prophylactic agent (mRNA) remains predominantly within the said organ. In some embodiments, the term "local region" or "locally" can mean that within 4-8 hours (e.g., 4 hours) after administration of the LNP composition described herein to a tissue (e.g., a muscle or bone), the LNP composition, the therapeutic and/or prophylactic agent (mRNA) remains predominantly within the said tissue; particularly, the LNP composition is not delivered to the liver within 4-8 hours (e.g., 4 hours) post-administration.

### Lipids and Lipid Nanoparticle Compositions

The lipids described herein can be advantageously used in lipid nanoparticle compositions to deliver a therapeutic and/or prophylactic agent locally to a mammal. The lipids described herein have little or no immunogenicity.

In some aspects, the compound described herein is of Formula (I) or a salt or isomer thereof. A lipid according to Formula (I) may have a positive or partial positive charge at physiological pH. Such lipids can be referred to as cationic lipids.

As used herein, the term "alkyl" or "alkyl group" refers to a linear or branched saturated hydrocarbon including one or more carbon atoms (e.g., 1, 2, 3, 4, 5, 6, 7, 8, 9, 10, 11, 12, 13, 14, 15, 16, 17, 18, 19, 20, or more carbon atoms), which is optionally substituted. The notation "C1-14 alkyl" refers to an optionally substituted linear or branched saturated hydrocarbon including 1-14 carbon atoms. Unless otherwise stated, an alkyl group as described herein refers to both unsubstituted and substituted alkyl group.

As used herein, the term "alkenyl" or "alkenyl group" refers to a linear or branched hydrocarbon including two or more carbon atoms (e.g., 2, 3, 4, 5, 6, 7, 8, 9, 10, 11, 12, 13, 14, 15, 16, 17, 18, 19, 20, or more carbon atoms) and at least one carbon-carbon double bond, which is optionally substituted. The notation "C2-14 alkenyl" refers to an optionally substituted linear or branched hydrocarbon including 2-14 carbon atoms and at least one carbon-carbon double bond. An alkenyl group may include 1, 2, 3, 4, or more carbon-carbon double bonds. Unless otherwise stated, an alkenyl group as described herein refers to both unsubstituted and substituted alkenyl group.

As used herein, the term "alkynyl" or "alkynyl group" refers to a linear or branched hydrocarbon including two or more carbon atoms (e.g., 2, 3, 4, 5, 6, 7, 8, 9, 10, 11, 12, 13, 14, 15, 16, 17, 18, 19, 20, or more carbon atoms) and at least one carbon-carbon triple bond, which is optionally substituted. The notation "C2-14 alkynyl" refers to an optionally substituted linear or branched hydrocarbon including 2-14 carbon atoms and at least one carbon-carbon triple bond. An alkynyl group may include 1, 2, 3, 5, or more carbon-carbon triple bonds. Unless otherwise stated, an alkynyl group as described herein refers to both unsubstituted and substituted alkynyl group.

As used herein, the term "carbocycle" or "carbocyclic group" refers to an optionally substituted mono- or multi-cyclic system comprising one or more rings of carbon atoms. A ring can be 3-, 4-, 5-, 6-, 7-, 8-, 9-, 10-, 11-, 12-, 13-, 14-, 15-, 16-, 17-, 18-, 19-, 20-, or more-membered. The notation "C3-6 carbocycle" refers to a carbocycle comprising a single ring having 3-6 carbon atoms. A carbocycle may include one or more carbon-carbon double bonds or triple bonds and may be non-aromatic or aromatic (e.g., cycloalkyl or aryl). Examples of carbocycles include cyclopropyl, cyclopentyl, cyclohexyl, phenyl, naphthyl.

As used herein, the term "cycloalkyl" refers to a non-aromatic carbocycle and may or may not include any double or triple bonds. Unless otherwise stated, carbocycles as described herein refer to both unsubstituted and substituted carbocyclic group, i.e., optionally substituted carbocycles.

As used herein, the term "heterocycle" or "heterocyclic group" refers to an optionally substituted mono- or multi-cyclic system including one or more rings, wherein at least one ring includes at least one heteroatom. Heteroatoms may be, for example, nitrogen, oxygen, or sulfur atoms. Rings can be 3-, 4-, 5-, 6-, 7-, 8-, 9-, 10-, 11-, 12-, 13-, 14-, or more membered rings. Heterocycles may include one or more double or triple bonds and may be non-aromatic or aromatic (e.g., heterocycloalkyl or heteroaryl). Examples of heterocycles include imidazolyl, imidazolidinyl, oxazolyl, oxazolidinyl, thiazolyl, thiazolidinyl, pyrazolidinyl, pyrazolyl, isoxazolidinyl, isoxazolyl, isothiazolidinyl, isothiazolyl, morpholinyl, pyrrolyl, pyrrolidinyl, furanyl, tetrahydrofuranyl, thiophenyl, pyridyl, piperidinyl, quinolinyl, and isoquinolinyl.

The term "heterocycloalkyl" as used herein refers to a non-aromatic heterocycle and may or may not include any double or triple bonds. Unless otherwise stated, heterocycles as described herein refer to both unsubstituted and substituted heterocyclic groups, i.e., optionally substituted heterocycles.

As used herein, the term "aryl" or "aryl group" is an optionally substituted carbocyclic group including one or more aromatic rings. Examples of aryl groups include phenyl group and naphthyl group.

As used herein, the term "heteroaryl" is an optionally substituted heterocyclic group including one or more aromatic rings. Examples of heteroaryls include pyrrolyl, furanyl, thiophenyl, imidazolyl, oxazolyl, and thiazolyl. Both aryl and heteroaryl may be optionally substituted. Unless otherwise stated, aryl groups or heteroaryl groups as described herein refer to both unsubstituted and substituted groups, i.e., optionally substituted aryl or heteroaryl groups.

Unless otherwise stated, alkyl, alkenyl, and cyclic groups (e.g., carbocyclic and heterocyclic groups) may be optionally substituted. Optional substituents may be selected from the group consisting of, but are not limited to, a halogen atom (e.g., a chloro, bromo, fluoro, or iodo group), a carboxylic acid (e.g., -C(O)OH), an alcohol (e.g., a hydroxyl, -OH), an ester (e.g., -C(O)OR or -OC(O)R), an aldehyde (e.g., - C(O)H), a carbonyl (e.g., -C(O)R, or denoted as C=O), an acyl halide (e.g., -C(O)X, where X is a halide selected from bromide, fluoride, chloride, and iodide), a carbonate (e.g., -OC(O)OR), an alkoxy (e.g., -OR), an acetal (e.g., -C(OR)₂R', where each OR is an alkoxy group which may be the same or different, and R' is an alkyl or alkenyl group), a phosphate (e.g., P(O)₄³⁺), a thiol (e.g., -SH), a sulfoxide (e.g., -S(O)R), a sulfinic acid (e.g., -S(O)OH), a sulfonic acid (e.g., -S(O)₂OH), a thioaldehyde (e.g., - C(S)H), a sulfate (e.g., S(O)₄²⁺), a sulfonyl (e.g., -S(O)₂-), an amide (e.g., -C(O)NR₂ or -N(R)C(O)R), an azido (e.g., -N₃), a nitro (e.g., -NO₂), a cyano (e.g., -CN), an isocyano (e.g., -NC), an acyloxy (e.g., -OC(O)R), an amino (e.g., -NR₂, -NRH, or - NH₂), a carbamoyl (e.g., -OC(O)NR₂, -OC(O)NRH, or -OC(O)NH₂), a sulfonamide (e.g., -S(O)₂NR₂, -S(O)₂NRH, -S(O)₂NH₂, -N(R)S(O)₂R, -N(H)S(O)₂R, - N(R)S(O)₂H, or -N(H)S(O)₂H), an alkyl, alkenyl, and cyclic group (e.g., a carbocyclic or heterocyclic group). In any of the preceding, R is an alkyl or alkenyl group as defined herein.

In some embodiments, the substituents themselves may be further substituted by, for example, 1, 2, 3, 4, 5, or 6 substituents as defined herein. For example, C1-6 alkyl may be further substituted by 1, 2, 3, 4, 5, or 6 substituents as described herein.

As used herein, the terms "about" and "approximately" when applied to one or more values of interest refer to a value similar to the stated reference value. In certain embodiments, the terms "about" or "approximately" mean within 25%, 20%, 19%, 18%, 17%, 16%, 15%, 14%, 13%, 12%, 11%, 10%, 9%, 8%, 7%, 6%, 5%, 4%, 3%, 2%, 1% or less in either direction (greater than or less than) of the stated reference value, unless otherwise stated or otherwise evident from the context (except where such number would exceed 100% of a possible value). For example, when used in the context of the amount of a given compound in a lipid component of a nanoparticle composition, "about" may mean +/-10% of the recited value. For example, a nanoparticle composition comprising a lipid component having about 40% of a given compound may include 30-50% of the compound.

As used herein, the term "compound" is intended to include all isomers and isotopes of the structure depicted. "Isotope" refers to an atom having the same atomic number but different mass numbers resulting from a different number of neutrons in the nucleus, e.g., isotopes of hydrogen include tritium and deuterium. Furthermore, a compound, salts, or complex of the present disclosure may be prepared in combination with solvent or water molecules to form solvates and hydrates by routine methods.

As used herein, the term "contact" means establishing a physical connection between two or more entities. For example, contacting a mammalian cell with a nanoparticle composition means establishing a physical connection between the mammalian cell and the nanoparticle composition. Methods of contacting cells with external entities *in vivo* and *in vitro* are well known in biological arts. For example, the contacting of nanoparticle compositions with mammalian cells can be achieved via different routes of administration (e.g., intravenous, intramuscular, intradermal, and subcutaneous) and may involve different amounts of the nanoparticle composition. Furthermore, a nanoparticle composition may contact more than one type of mammalian cell.

As used herein, the term "delivery" means providing an entity to a destination. For example, delivering a therapeutic and/or prophylactic agent to a subject may involve administering to the subject a nanoparticle composition comprising the therapeutic and/or prophylactic agent (e.g., via intravenous, intramuscular, intradermal, or subcutaneous routes). Administering a nanoparticle composition to a mammal or mammalian cells may involve contacting one or more cells with the nanoparticle composition. "Local delivery" or "locally delivering" means delivering a medicament primarily to a local region of the subject, such as an organ, a tissue (e.g., a muscle), or intracellular cells (e.g., the organ, muscle, or cells to which the medicament is administered). Organs, tissues, or cells other than said organ, tissue, or cells are not delivered to or are essentially not delivered to. For example, when a medicament is administered into a kidney, the medicament is delivered locally to other regions within the kidney, and is not delivered to organs, tissues, or cells other than the kidney (e.g., via a circulatory system). When a medicament is administered into a muscle (e.g., a gastrocnemius), the medicament is delivered locally to other regions within the muscle, and is not delivered to organs, tissues, or cells other than the muscle (e.g., via a circulatory system).

In certain embodiments, nanoparticle compositions comprising a compound according to Formula (I) have substantially the same level of local delivery effect, regardless of the route of administration. For example, when certain compounds disclosed herein are used for intravenous or intramuscular delivery of a therapeutic and/or prophylactic agent, they exhibit similar local delivery effect. In some embodiments, intramuscular injection is superior to intravenous injection.

As used herein, the terms "specific delivery", "local delivery", "locally delivering" mean that, compared to non-target tissues (e.g., mammalian livers), a greater amount (e.g., at least 1.5-fold more, at least 2-fold more, at least 3-fold more, at least 4-fold more, at least 5-fold more, at least 6-fold more, at least 7-fold more, at least 8-fold more, at least 9-fold more, at least 10-fold more) of a therapeutic and/or prophylactic agent is delivered via nanoparticles to the target cells, tissue, or organ of interest (e.g., mammalian muscles). The level of nanoparticle delivery to a specific tissue can be identified by comparing the following: the amount of protein produced in a tissue to the weight of the tissue, the amount of the therapeutic and/or prophylactic agent in a tissue to the weight of the tissue, the amount of protein produced in a tissue to the total amount of protein in the tissue, or the amount of the therapeutic and/or prophylactic agent in a tissue to the total amount of the therapeutic and/or prophylactic agent in the tissue. It should be understood that the ability of nanoparticles to deliver specifically to a target tissue need not be determined in a subject receiving treatment; it can be determined in a surrogate such as an animal model (e.g., a rat model).

As used herein, "encapsulation efficiency" refers to the amount of the therapeutic and/or prophylactic agent that becomes part of a nanoparticle composition relative to the initial total amount of the therapeutic and/or prophylactic agent used to prepare the nanoparticle composition. For example, if 97 mg of a therapeutic and/or prophylactic agent out of a total of 100 mg initially provided to the composition is encapsulated in the nanoparticle composition, the encapsulation efficiency can be 97%.

As used herein, "expression" of a nucleic acid sequence includes translation of mRNA into a polypeptide or protein and/or post-translational modification of the polypeptide or protein.

As used herein, the term "in vitro" refers to events that occur in an artificial environment, such as in a test tube or reaction vessel, in a cell culture, in a Petri dish, etc., rather than within a living organism (e.g., an animal, plant, or microorganism). As used herein, the term "in vivo" refers to events that occur within a living organism (e.g., an animal, plant, or microorganism, or a cell or tissue thereof). As used herein, the term "ex vivo" refers to events that occur outside a living organism (e.g., an animal, plant, or microorganism, or a cell or tissues thereof). Ex vivo events may occur in an environment with minimal alteration compared to a natural (e.g., in vivo) environment.

As used herein, the term "isomer" refers to any geometric isomer, tautomer, zwitterion, stereoisomer, enantiomer, or diastereomer of a compound. Compounds may include one or more chiral centers and/or double bonds and thus may exist as stereoisomers. The present disclosure encompasses any and all isomers of the compounds described herein, including stereoisomerically pure forms as well as mixtures of enantiomers and stereoisomers, e.g., racemates. Methods of preparing enantiomeric and stereoisomeric mixtures of compounds and resolving them into their enantiomeric or stereoisomeric components are well known.

As used herein, a "lipid component" is a component of a nanoparticle composition comprising one or more lipids. For example, a lipid component may include one or more cationic/ionizable, PEGylated, structural, or other lipids, such as phospholipids.

As used herein, "administering" may include intravenous, intramuscular, intradermal, subcutaneous, or other methods of delivering a composition to a subject. The method of administration can be chosen to target delivery (e.g., specific delivery) to a particular area or system of the body.

As used herein, "modified" means non-natural. For example, an RNA can be a modified RNA. That is, the RNA can include one or more non-naturally occurring nucleobases, nucleosides, nucleotides, or linkers. An RNA also includes any sequence optimization performed on the RNA.

As used herein, a "nanoparticle composition" is a composition comprising one or more lipids. Nanoparticle compositions are typically micron-sized or smaller and may include a lipid bilayer. Nanoparticle compositions include lipid nanoparticles (LNPs), liposomes (e.g., lipid vesicles), and lipid complexes. For example, a nanoparticle composition can be a liposome with a lipid bilayer having a diameter of 500 nm or less.

As used herein, a "patient" refers to a subject who may seek or need treatment, requires treatment, is receiving treatment, will receive treatment, or has received care from a trained professional for a specific disease or disorder.

As used herein, "PEG lipid" or "PEGylated lipid" refers to a lipid comprising a polyethylene glycol component.

The phrase "pharmaceutically acceptable" as used herein refers to those compounds, materials, compositions, and/or dosage forms that are, within the scope of sound medical judgment, suitable for contact with human and animal tissues without excessive toxicity, irritation, allergic response, or other problems or complications, commensurate with a reasonable benefit/risk ratio.

As used herein, the phrase "pharmaceutically acceptable excipient" means any ingredient other than the compound described herein (e.g., a carrier capable of suspending, complexing, or dissolving the active compound) and having substantially non-toxic and harmless properties. Excipients may include, for example: antiadherents, antioxidants, binders, coatings, compression aids, disintegrants, dyes (pigments), emollients, emulsifiers, fillers (diluents), film-forming agents or coating agents, fragrances, flavorings, glidants (flow enhancers), lubricants, preservatives, printing inks, sorbents, suspending or dispersing agents, sweeteners, and water of hydration. Exemplary excipients include but are not limited to: butylated hydroxytoluene (BHT), calcium carbonate, calcium phosphate (dibasic), calcium stearate, croscarmellose sodium, crospovidone, citric acid, crospovidone, cysteine, ethyl cellulose, gelatin, hydroxypropyl cellulose, hydroxypropyl methylcellulose, lactose, magnesium stearate, maltitol, mannitol, methionine, methylcellulose, methylparaben, microcrystalline cellulose, polyethylene glycol, polyvinylpyrrolidone, povidone, pregelatinized starch, propylparaben, retinyl palmitate, shellac, silica, sodium carboxymethylcellulose, sodium citrate, sodium starch glycolate, sorbitol, starch (corn), stearic acid, sucrose, talc, titanium dioxide, vitamin A, vitamin E (α-tocopherol), vitamin C, xylitol, and others disclosed herein.

In this specification, structural formulas of compounds in certain cases are drawn for convenience to represent a specific isomer, but the present disclosure includes all isomers, such as geometric isomers, optical isomers based on asymmetric carbons, stereoisomers, tautomers, etc., it being understood that not all isomers may have the same level of activity.

For compounds represented by the chemical formulas herein, crystalline polymorphism may exist. It should be noted that any crystal form, mixture of crystal forms, or anhydrides or hydrates thereof are included within the scope of the present disclosure. The term "crystalline polymorph", "polymorph", or "crystal form" refers to a crystal structure in which a compound (or its salt or solvate) can crystallize in different crystal packing arrangements, all having the same elemental composition. Different crystal forms typically have different X-ray diffraction patterns, infrared spectra, melting points, densities, hardness, crystal shapes, optical and electrical properties, stabilities, and solubilities. Recrystallization solvent, crystallization rate, storage temperature, and other factors may cause one crystal form to predominate. Crystalline polymorphs of compounds can be prepared by crystallization under different conditions.

Compositions may also include salts of one or more compounds. The salts may be pharmaceutically acceptable salts. As used herein, "pharmaceutically acceptable salts" refers to derivatives of the disclosed compounds wherein the parent compound is altered by converting an existing acid or base moiety to its salt form (e.g., by reacting a free base with a suitable organic acid). Examples of pharmaceutically acceptable salts include, but are not limited to, inorganic or organic acid salts of basic residues such as amino groups; alkali metal or organic salts of acidic residues such as carboxylic acids, etc.

Representative acid addition salts include acetate, adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, fumarate, glucoheptonate, glycerophosphate, hemisulfate, heptanoate, hexanoate, hydrobromide, hydrochloride, hydroiodide, 2-hydroxyethanesulfonate, lactobionate, lactate, laurate, lauryl sulfate, maleate, malate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, toluenesulfonate, undecanoate, valerate, and the like.

Representative alkali metal or alkaline earth metal salts include sodium, lithium, potassium, calcium, magnesium, and the like, as well as non-toxic ammonium, quaternary ammonium, and amine cations, including but not limited to ammonium, tetramethylammonium, tetraethylammonium, methylamine, dimethylamine, trimethylamine, triethylamine, ethylamine, and the like.

Pharmaceutically acceptable salts of the present disclosure include, for example, conventional non-toxic salts of the parent compound formed from non-toxic inorganic or organic acids.

Pharmaceutically acceptable salts of the present disclosure can be synthesized from the parent compound containing an acidic or basic moiety by conventional chemical methods. Generally, such salts can be prepared by reacting the free acid or base forms of these compounds with a stoichiometric amount of the appropriate base or acid in water or an organic solvent or in a mixture of the two; typically, nonaqueous media such as ether, ethyl acetate, ethanol, isopropanol, or acetonitrile are preferred. A list of suitable salts can be found in any edition of *Remington's Pharmaceutical Sciences*, which is incorporated herein by reference in its entirety.

As used herein, a "phospholipid" is a lipid comprising a phosphate moiety and one or more carbon chains, e.g., unsaturated fatty acid chains. A phospholipid may include one or multiple (e.g., double or triple) bonds (e.g., one or more unsaturations). Specific phospholipids may facilitate fusion with a membrane. For example, cationic phospholipids can interact with one or more negatively charged phospholipids of a membrane (e.g., a cellular or intracellular membrane). Fusion of a phospholipid with a membrane may allow one or more components of a lipid-containing composition to pass through the membrane permitting, e.g., delivery of one or more components to a cell.

As used herein, the term "polypeptide" or "polypeptide of interest" refers to a polymer of amino acid residues typically linked by peptide bonds, which may be produced naturally (e.g., isolated or purified) or synthetically.

As used herein, an "RNA" refers to a ribonucleic acid that may be naturally or non-naturally occurring. For example, an RNA may include modified and/or non-naturally occurring components, such as one or more nucleobases, nucleosides, nucleotides, or linkers. An RNA may include a cap structure, a chain-terminating nucleotide, a stem-loop, a polyA sequence, and/or a polyadenylation signal. An RNA may have a nucleotide sequence encoding a polypeptide of interest. For example, an RNA may be a messenger RNA (mRNA). Translation of an mRNA encoding a specific polypeptide, e.g., *in vivo* translation of an mRNA inside a mammalian cell, can produce the encoded polypeptide. RNAs may be selected from small interfering RNA (siRNA), asymmetric interfering RNA (aiRNA), microRNA (miRNA), Dicer-substrate RNA (dsRNA), small hairpin RNA (shRNA), and mRNA.

As used herein, a "single unit dose" is a dose of any therapeutic administered in one dose/at one time/single route/single point of contact, i.e., single administration event. As used herein, a "divided dose" refers to dividing a single unit dose or total daily dose into two or more doses. As used herein, a "total daily dose" is an amount given or prescribed in a 24-hour period. It may be administered as a single unit dose.

As used herein, "size" or "average size" in the context of a nanoparticle composition refers to the mean diameter of the nanoparticle composition.

As used herein, the terms "subject", "individual", or "patient" refer to any organism to which a composition according to the present disclosure may be administered, e.g., for experimental, diagnostic, prophylactic, and/or therapeutic purposes. Typical subjects include animals (e.g., mammals such as mice, rats, rabbits, non-human primates, and humans) and/or plants. Preferably, the subject described herein suffers from a disease that benefits from local delivery of a medicament, e.g., a lesion in an organ or tissue where it is desired to deliver the medicament only to that organ or tissue.

As used herein, "target cells" refers to any one or more cells of interest. The cells may be found *in vitro*, *in vivo*, *in situ*, or in the tissue or organ of an organism. The organism may be an animal, preferably a mammal, more preferably a human, and most preferably a patient.

As used herein, "target tissue" refers to any one or more tissue types of interest where delivery of a therapeutic and/or prophylactic agent will result in a desired biological and/or pharmacological effect. Examples of target tissues of interest include specific tissues, organs, and systems or groups thereof. In specific applications, the target tissue may be a kidney, lung, spleen, vascular endothelium (e.g., intracoronary or intrafemoral), or tumor tissue (e.g., via intratumoral injection) or muscle (e.g., via intramuscular injection).

As used herein, "muscle" includes but is not limited to: back muscles (including trapezius, latissimus dorsi, levator scapulae, rhomboids, erector spinae, splenius, transversospinalis, interspinales, intertransversarii), chest muscles (including pectoralis major, pectoralis minor, serratus anterior, external intercostals, internal intercostals, transversus thoracis), diaphragm, abdominal muscles (including rectus abdominis, external oblique, internal oblique, transversus abdominis, quadratus lumborum), perineal muscles, shoulder girdle muscles (including deltoid, supraspinatus, infraspinatus, teres minor, subscapularis, teres major), arm muscles (including biceps brachii, coracobrachialis, brachialis, triceps brachii, anconeus), forearm muscles (including brachioradialis, pronator teres, flexor carpi radialis, flexor carpi ulnaris, extensor carpi radialis longus, extensor carpi radialis brevis, extensor carpi ulnaris), hand muscles, pelvic girdle muscles (including iliopsoas, piriformis, gluteus maximus, gluteus medius, gluteus minimus), thigh muscles (including quadriceps femoris, sartorius, tensor fasciae latae, biceps femoris, semitendinosus, semimembranosus, pectineus, adductor longus, adductor brevis, adductor magnus, gracilis), leg muscles (including tibialis anterior, extensor digitorum longus, gastrocnemius, soleus, flexor digitorum longus, flexor hallucis longus, tibialis posterior, peroneus longus and peroneus brevis), foot muscles, platysma, and sternocleidomastoid.

The term "therapeutic agent" or "prophylactic agent" refers to any agent that has a therapeutic, diagnostic, and/or prophylactic effect and/or elicits a desired biological and/or pharmacological effect when administered to a subject. Therapeutic agents are also called "active agents" and include, but are not limited to, cytotoxins, radioactive ions, chemotherapeutic medicaments, small molecule medicaments, proteins, and nucleic acids such as RNA.

As used herein, the term "therapeutically effective amount" refers to an amount that, when administered to a subject suffering from or susceptible to an infection, disease, disorder, and/or condition, treats, ameliorates symptoms of, diagnoses, prevents, and/or delays the onset of the infection, disease, disorder, and/or condition.

As used herein, "transfection" means introducing a substance (e.g., RNA) into a cell. Transfection can occur, for example in vitro, ex vivo, or in vivo.

As used herein, the term "treat", "treating" or "treatment" refers to partially or completely alleviating, ameliorating, improving, relieving, delaying the onset of, inhibiting the progression of, reducing the severity of, and/or reducing the incidence of a specific infection, disease, disorder, and/or condition. For example, "treating" cancer may refer to inhibiting the survival, growth, and/or spread of a tumor. Subjects who do not exhibit signs of a disease, disorder, and/or condition and/or subjects who exhibit only early signs of a disease, disorder, and/or condition may be treated to reduce the risk of developing pathology associated with the disease, disorder, and/or condition.

Another aspect of the present disclosure relates to a nanoparticle composition comprising a lipid component, wherein the lipid component comprises a compound of Formula (I) as described herein.

Nanoparticle compositions include, for example, lipid nanoparticles (LNPs), liposomes, lipid vesicles, and lipid complexes. In some embodiments, the nanoparticle composition is a vesicle comprising one or more lipid bilayers. In certain embodiments, the nanoparticle composition comprises two or more concentric bilayers separated by aqueous compartments. The lipid bilayers may be functionalized and/or cross-linked to each other. The lipid bilayers may include one or more ligands, proteins, or channels.

The nanoparticle composition comprises a lipid component, which includes at least one compound according to Formula (I). For example, the lipid component of the nanoparticle composition may include one or more of Compounds 1-10. The nanoparticle composition may also include a variety of other components. For example, in addition to the lipid of Formula (I), the nanoparticle composition may include one or more cationic lipids.

The lipid component of the nanoparticle composition may include one or more PEG or PEG-modified lipids. Such substances may alternatively be referred to as PEGylated lipids. A PEG lipid is a lipid modified with polyethylene glycol. The PEG lipid may be selected from PEG-modified phosphatidylethanolamines, PEG-modified phosphatidic acids, PEG-modified ceramides, PEG-modified dialkylamines, PEG-modified diacylglycerols, PEG-modified dialkylglycerols, and mixtures thereof. For example, the PEG lipid may be PEG-c-DOMG, PEG-DMG, PEG-DLPE, PEG-DMPE, PEG-DPPC, or PEG-DSPE lipid.

The lipid component of the nanoparticle composition may include one or more structural lipids. The structural lipid may be selected from, but not limited to, cholesterol, coprostanol, sitosterol, ergosterol, campesterol, stigmasterol, brassicasterol, tomatidine, tomatine, ursolic acid, α-tocopherol, and mixtures thereof. In some embodiments, the structural lipid is cholesterol. In some embodiments, the structural lipid comprises cholesterol and a corticosteroid (e.g., prednisolone, dexamethasone, prednisone, and hydrocortisone) or a combination thereof.

The lipid component of the nanoparticle composition may include one or more phospholipids, e.g., one or more unsaturated lipids. The phospholipid(s) may assemble into one or more lipid bilayers. Typically, a phospholipid may include a phospholipid moiety and one or more fatty acid moieties. For example, the phospholipid may be a lipid according to Formula (II): 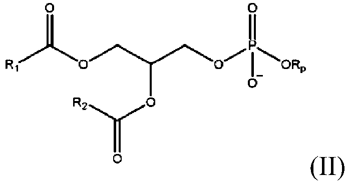 wherein Rp represents a phosphatidyl moiety, and R1 and R2 represent fatty acid moieties with or without unsaturation, which may be the same or different. The phosphatidyl moiety may be selected from phosphatidylcholine, phosphatidylethanolamine, phosphatidylglycerol, phosphatidylserine, phosphatidic acid, 2-lysophosphatidylcholine, and sphingomyelin. The fatty acid moieties may be selected from lauric acid, myristic acid, myristoleic acid, palmitic acid, palmitoleic acid, stearic acid, oleic acid, linoleic acid, alpha-linolenic acid, erucic acid, phytanic acid, arachidic acid, arachidonic acid, eicosapentaenoic acid, behenic acid, docosapentaenoic acid, and docosahexaenoic acid.

Phospholipids suitable for use in the compositions and methods described herein may be selected from DSPC, DOPE, DLPC, DMPC, DOPC, DPPC, DUPC, POPC, OChemsPC, DOPG, and sphingomyelin. In some embodiments, the nanoparticle composition comprises DSPC. In some embodiments, the nanoparticle composition comprises DOPE. In some embodiments, the nanoparticle composition comprises DSPC and/or DOPE.

In some embodiments, the nanoparticle composition described herein comprises a compound of Formula (I), a PEG lipid, DSPC, and cholesterol.

In some embodiments, nanoparticle compositions comprising one or more lipids as described herein may further comprise one or more adjuvants, such as aluminum hydroxide.

### Therapeutic and/or Prophylactic Agents

The nanoparticle composition may comprise one or more therapeutic and/or prophylactic agents. Methods of the present disclosure for delivering a therapeutic and/or prophylactic agent to a mammalian cell or organ, producing a polypeptide of interest in a mammalian cell, and treating a disease or disorder in a mammal in need thereof, comprise administering and/or contacting mammalian cells with a nanoparticle composition comprising the therapeutic and/or prophylactic agent.

The therapeutic and/or prophylactic agent may be a substance that, once delivered to a cell or organ, brings about a desired change in the cell, organ, or other body tissue or system. Such species may be used to treat one or more diseases, disorders, or conditions. In some embodiments, the therapeutic and/or prophylactic agent is a small molecule medicament useful in treating a specific disease, disorder, or condition.

Examples of agents suitable for use in nanoparticle compositions include, but are not limited to, antineoplastic agents (e.g., doxorubicin, mitoxantrone, camptothecin, cisplatin, bleomycin, cyclophosphamide, streptozotocin, actinomycin D, vincristine, vinblastine, cystine arabinoside, anthracyclines, alkylating agents, platinum compounds, antimetabolites, and nucleoside analogs such as methotrexate and purine and pyrimidine analogs), anti-infective agents, local anesthetics (e.g., dibucaine and chlorpromazine), β-adrenergic blockers (e.g., propranolol, timolol, and labetalol), antihypertensive agents (e.g., clonidine and hydralazine), antidepressants (e.g., imipramine, amitriptyline, and doxepin), antiepileptics (e.g., phenytoin), antihistamines (e.g., diphenhydramine, chlorpheniramine, and promethazine), antibiotics/antibacterials (e.g., gentamicin, ciprofloxacin, and cefoxitin), antifungals (e.g., miconazole, terconazole, econazole, isoconazole, butoconazole, clotrimazole, itraconazole, nystatin, naftifine, and amphotericin B), antiparasitic agents, hormones, hormone antagonists, immunomodulators, neurotransmitter antagonists, anti-glaucoma agents, vitamins, anesthetics, and imaging agents.

In some embodiments, the therapeutic and/or prophylactic agent is a cytotoxin, radioactive ion, chemotherapeutic agent, vaccine, compound that elicits an immune response, and/or another therapeutic and/or prophylactic agent. Cytotoxins or cytotoxic agents include any agent that may be detrimental to cells. Examples include but are not limited to paclitaxel, cytochalasin B, gramicidin D, ethidium bromide, emetine, mitomycin, etoposide, teniposide, vincristine, vinblastine, colchicine, doxorubicin, daunorubicin, dihydroxyanthracenedione, mitoxantrone, mithramycin, actinomycin D, 1-dehydrotestosterone, glucocorticoids, procaine, tetracaine, lidocaine, propranolol, puromycin, maytansinoids, e.g., maytansinol, racemomycin, and analogs or homologs thereof. Radioactive ions include but are not limited to iodine (e.g., iodine-125 or iodine-131), strontium-89, phosphorus, palladium, cesium, iridium, phosphate, cobalt, yttrium-90, samarium-153, and praseodymium.

Other therapeutic and/or prophylactic agents include but are not limited to antimetabolites (e.g., methotrexate, 6-mercaptopurine, 6-thioguanine, cytarabine, 5-fluorouracil decarbazine), alkylating agents (e.g., nitrogen mustards, thiotepa, chlorambucil, racemomycin, melphalan, carmustine (BSNU), lomustine (CCNU), cyclophosphamide, busulfan, dibromomannitol, streptozotocin, mitomycin C, and cis-diamminedichloroplatinum(II) (DDP) cisplatin), anthracyclines (e.g., daunorubicin (formerly daunomycin) and doxorubicin), antibiotics (e.g., dactinomycin (formerly actinomycin), bleomycin, mithramycin, and anthramycin (AMC)), and antimitotic agents (e.g., vincristine, vinblastine, paclitaxel, and maytansinoids).

In other embodiments, the therapeutic and/or prophylactic agent is a protein. Therapeutic proteins useful in the nanoparticles of the present disclosure include, but are not limited to, gentamicin, amikacin, insulin, erythropoietin (EPO), granulocyte colony-stimulating factor (G-CSF), granulocyte-macrophage colony-stimulating factor (GM-CSF), Factor VIR, luteinizing hormone-releasing hormone (LHRH) analogs, interferons, heparin, hepatitis B surface antigen, typhoid vaccine, and cholera vaccine.

In some embodiments, the therapeutic agent is a polynucleotide or nucleic acid (e.g., ribonucleic acid or deoxyribonucleic acid). The term "polynucleotide", in its broadest sense, includes any compound and/or substance that is or can be incorporated into an oligonucleotide chain. Exemplary polynucleotides for use according to the present disclosure include, but are not limited to, one or more of deoxyribonucleic acid (DNA), ribonucleic acid (RNA) including messenger RNA (mRNA), hybrids thereof, RNAi inducers, RNAi agents, siRNA, shRNA, miRNA, antisense RNA, ribozymes, catalytic DNA, RNA inducing triple helix formation, aptamers, vectors, and the like.

In some embodiments, the therapeutic and/or prophylactic agent is an RNA. RNAs suitable for use in the compositions and methods described herein may be selected from, but not limited to, short-chain, antagonists, antisense strands, ribozymes, small interfering RNA (siRNA), asymmetric interfering RNA (aiRNA), microRNA (miRNA), Dicer-substrate RNA, small hairpin RNA (shRNA), transfer RNA (tRNA), messenger RNA (mRNA), and mixtures thereof. In certain embodiments, the RNA is an mRNA or siRNA.

In certain embodiments, the therapeutic and/or prophylactic agent is an mRNA. The mRNA can encode any polypeptide of interest, including any naturally or non-naturally occurring or otherwise modified polypeptide. The polypeptide encoded by the mRNA can be of any size and may have any secondary structure or activity. In some embodiments, the polypeptide encoded by the mRNA may have a therapeutic effect when expressed in a cell.

In other embodiments, the therapeutic and/or prophylactic agent is an siRNA. siRNA may be capable of selectively knocking down or down-regulating expression of a gene of interest. For example, siRNA may be selected to silence a gene associated with a specific disease or disorder when a nanoparticle composition comprising the siRNA is administered to a subject in need thereof. The siRNA may comprise a sequence complementary to an mRNA sequence encoding the gene or protein of interest. In some embodiments, the siRNA may be an immunomodulatory siRNA.

In some embodiments, the therapeutic and/or prophylactic agent is shRNA or a vector or plasmid encoding the same. shRNA can be produced within a target cell after delivery of an appropriate construct to the nucleus. Constructs and mechanisms associated with shRNA are well known in the relevant art.

Nucleic acids and polynucleotides for use in the present disclosure typically comprise a first region (e.g., a coding region) of linked nucleotides encoding a polypeptide of interest, a first flanking region (e.g., 5'-UTR) located at the 5'-end of the first region, a second flanking region (e.g., 3'-UTR) located at the 3'-end of the first region, at least one 5'-cap region, and a 3'-stabilizing region. In some embodiments, the nucleic acid or polynucleotide further comprises a poly-A region or a Kozak sequence (e.g., in the 5'-UTR). In some cases, the polynucleotide may comprise one or more intron nucleotide sequences capable of being excised from the polynucleotide. In some embodiments, the polynucleotide or nucleic acid (e.g., an mRNA) may include a 5' cap structure, chain-terminating nucleotides, stem-loops, polyadenylic acid sequences, and/or polyadenylation signals. Any one region of the nucleic acid may include one or more alternative components (e.g., alternative nucleotides).

Typically, the shortest length of the polynucleotide can be a length sufficient to encode a dipeptide. In another embodiment, the length of the polynucleotide sequence is sufficient to encode a tripeptide, tetrapeptide, pentapeptide, hexapeptide, heptapeptide, octapeptide, nonapeptide, or decapeptide. In some cases, the polynucleotide is at least 30, at least 35, at least 40, at least 45, at least 50, at least 55, at least 60, at least 80, at least 90, at least 100, at least 120, at least 150, at least 180, at least 200, at least 300, at least 400, at least 500, at least 600, at least 700, at least 800, at least 900, at least 1000, at least 2000, at least 3000, at least 4000, or at least 5000 nucleotides in length.

Nucleic acids and polynucleotides may include one or more naturally occurring components, including any typical nucleotide A (adenosine), G (guanosine), C (cytidine), U (uridine), or T (thymidine). In one embodiment, all or substantially all nucleotides comprise (a) a 5'-UTR, (b) an open reading frame (ORF), (c) a 3'-UTR, (d) a polyadenylate tail, and any combination of the above a, b, c, or d.

Polynucleotides and nucleic acids may include one or more modified (e.g., altered or alternative) nucleobases, nucleosides, nucleotides, or combinations thereof. Nucleic acids and polynucleotides for use in nanoparticle compositions may include any useful modification or alteration, for example to the nucleobase, sugar, or internucleoside linkage (e.g., linking phosphate/phosphodiester bond/phosphodiester backbone).

In some cases, the nucleic acid is substantially non-immunostimulatory in cells into which the polynucleotide (e.g., an mRNA) is introduced.

### Other Components

In addition to those described in the preceding sections, the nanoparticle composition may include one or more other components. For example, the nanoparticle composition may include one or more hydrophobic small molecules, such as vitamins (e.g., vitamin A or vitamin E) or sterols.

The nanoparticle composition may also include one or more permeability enhancer molecules, carbohydrates, polymers, surface-modifying agents, or other components. Permeability enhancer molecules can be, for example, molecules described in U.S. Patent Application Publication No. 2005/0222064. Carbohydrates may include monosaccharides (e.g., glucose) and polysaccharides (e.g., glycogen and derivatives and analogs thereof). Polymers may be included in and/or used to encapsulate or partially encapsulate the nanoparticle composition. The polymer may be biodegradable and/or biocompatible. The polymer may be selected from, but not limited to, polyamines, polyethers, polyamides, polyesters, polyurethanes, polyureas, polycarbonates, polystyrenes, polyimides, polysulfones, polyacetylenes, polyethylenes, polyethylenimines, polyisocyanates, polyacrylates, polymethacrylates, polyacrylonitriles, and polyarylates. Surface-modifying agents may include, but are not limited to, anionic proteins (e.g., bovine serum albumin), surfactants (e.g., cationic surfactants such as dimethyldioctadecylammonium bromide), sugars or sugar derivatives (e.g., cyclodextrins), nucleic acids, polymers (e.g., heparin, polyethylene glycol, and poloxamers), mucolytics (e.g., acetylcysteine, neltenexine, and erdosteine), and DNase (e.g., rhDNase). The surface-modifying agent may be arranged within the nanoparticle and/or on the surface of the nanoparticle composition (e.g., by coating, adsorption, covalent linkage, or other processes).

The nanoparticle composition may further comprise one or more functionalized lipids. For example, a lipid may be functionalized with an alkyne group, which, when exposed to an azide under suitable reaction conditions, may undergo a cycloaddition reaction. In particular, the lipid bilayer may be functionalized in this way with one or more groups useful for promoting membrane penetration, cell recognition, or imaging.

The surface of the nanoparticle composition may also be conjugated with one or more useful antibodies. Functional groups and conjugates for targeted cell delivery, imaging, and membrane penetration are well known in the art.

In addition to these components, the nanoparticle composition may include any substance useful in a pharmaceutical composition. For example, the nanoparticle composition may include one or more pharmaceutically acceptable excipients or auxiliary ingredients, such as but not limited to one or more solvents, dispersion media, diluents, dispersion aids, suspension aids, granulation aids, disintegrants, fillers, glidants, liquid carriers, binders, surfactants, isotonic agents, thickeners or emulsifiers, buffers, lubricants, oils, preservatives, and other substances. Excipients such as waxes, butters, colorants, coating agents, flavorings, and fragrances may also be included. Pharmaceutically acceptable excipients are well known in the art (see, e.g., Remington's The Science and Practice of Pharmacy, 21st Edition).

The lipid component of the nanoparticle composition may include, for example, a lipid according to Formula (I), a phospholipid (e.g., an unsaturated lipid such as DOPE or DSPC), a PEG lipid, and a structural lipid. Each lipid component may be provided in specific fractions. In certain embodiments, the lipid component of the nanoparticle composition comprises about 30 mol% to about 60 mol% of the compound of Formula (I), about 0 mol% to about 30 mol% phospholipid, about 18.5 mol% to about 48.5 mol% structural lipid, and about 0 mol% to about 10 mol% PEG lipid. In some embodiments, the lipid component of the nanoparticle composition comprises about 35 mol% to about 55 mol% of the compound of Formula (I), about 5 mol% to about 25 mol% phospholipid, about 30 mol% to about 40 mol% structural lipid, and about 0 mol% to about 10 mol% PEG lipid. In specific embodiments, the lipid component comprises about 50 mol% of the compound of Formula (I), about 10 mol% phospholipid, about 38.5 mol% structural lipid, and about 1.5 mol% PEG lipid. In another specific embodiment, the lipid component comprises about 40 mol% of the compound of Formula (I), about 20 mol% phospholipid, about 38.5 mol% structural lipid, and about 1.5 mol% PEG lipid. In some embodiments, the phospholipid may be DOPE or DSPC. In other embodiments, the PEG lipid may be PEG-DMG and/or the structural lipid may be cholesterol.

The amount of the therapeutic and/or prophylactic agent in the nanoparticle composition may depend on the size, composition, desired target and/or application, or other properties of the nanoparticle composition as well as the nature of the therapeutic and/or prophylactic agent. For example, the amount of RNA suitable for use in the nanoparticle composition may depend on the size, sequence, and other features of the RNA. The relative amounts of the therapeutic and/or prophylactic agent and other components (e.g., lipids) in the nanoparticle composition may also vary. In some embodiments, the weight/weight ratio of the lipid component to the therapeutic and/or prophylactic agent in the nanoparticle composition may be from about 5:1 to about 60:1, e.g., 5:1, 6:1, 7:1, 8:1, 9:1, 10:1, 11:1, 12:1, 13:1, 14:1, 15:1, 16:1, 17:1, 18:1, 19:1, 20:1, 25:1, 30:1, 35:1, 40:1, 45:1, 50:1, 60:1. For example, the weight/weight ratio of the lipid component to the therapeutic and/or prophylactic agent may be from about 10:1 to about 40:1. In certain embodiments, the weight/weight ratio is about 20:1. The amount of the therapeutic and/or prophylactic agent in the nanoparticle composition can be measured, for example, using absorption spectroscopy (e.g., UV-Vis spectroscopy).

The average size of the nanoparticle composition may be between 10 nanometers and 100 nanometers, e.g., as measured by methods well known in the art. For example, the average size may be from about 40 nm to about 150 nm, e.g., about 40 nm, 45 nm, 50 nm, 55 nm, 60 nm, 65 nm, 70 nm, 75 nm, 80 nm, 85 nm, 90 nm, 95 nm, 100 nm, 105 nm, 110 nm, 115 nm, 120 nm, 125 nm, 130 nm, 135 nm, 140 nm, 145 nm, or 150 nm. In some embodiments, the average size of the nanoparticle composition may be from about 50 nm to about 100 nm, about 50 nm to about 90 nm, about 50 nm to about 80 nm, about 50 nm to about 70 nm, about 50 nm to about 60 nm, about 60 nm to about 100 nm, about 60 nm to about 90 nm, about 60 nm to about 80 nm, about 60 nm to about 70 nm, about 70 nm to about 100 nm, about 70 nm to about 90 nm, about 70 nm to about 80 nm, about 80 nm to about 100 nm, about 80 nm to about 90 nm, or about 90 nm to about 100 nm. In certain embodiments, the average size of the nanoparticle composition may be from about 70 nm to about 100 nm. In a specific embodiment, the average size may be about 70 nm.

The nanoparticle composition may be relatively homogeneous. The polydispersity index may be used to indicate the homogeneity of the nanoparticle composition, e.g., the particle size distribution of the nanoparticle composition. A small (e.g., less than 0.3) polydispersity index generally indicates a narrow particle size distribution. The nanoparticle composition may have a polydispersity index from about 0 to about 0.25, e.g., 0.01, 0.02, 0.03, 0.04, 0.05, 0.06, 0.07, 0.08, 0.09, 0.10, 0.11, 0.12, 0.13, 0.14, 0.15, 0.16, 0.17, 0.18, 0.19, 0.20, 0.21, 0.22, 0.23, 0.24, or 0.25. In some embodiments, the polydispersity index of the nanoparticle composition may be from about 0.05 to about 0.10.

The encapsulation efficiency of the therapeutic and/or prophylactic agent describes the amount of the therapeutic and/or prophylactic agent that is encapsulated or otherwise associated with the nanoparticle composition after preparation relative to the initial amount provided. The encapsulation efficiency may be measured, for example, by comparing the amount of the therapeutic and/or prophylactic agent in a solution containing the nanoparticle composition before and after breaking the nanoparticle composition with one or more organic solvents or detergents. Fluorescence can be used to measure the amount of free therapeutic and/or prophylactic agent (e.g., RNA) in solution. For the nanoparticle compositions described herein, the encapsulation efficiency of the therapeutic and/or prophylactic agent may be at least 50%, e.g., 50%, 55%, 60%, 65%, 70%, 75%, 80%, 85%, 90%, 91%, 92%, 93%, 94%, 95%, 96%, 97%, 98%, 99%, or 100%. In some embodiments, the encapsulation efficiency may be at least 80%. In certain embodiments, the encapsulation efficiency may be at least 90%.

The nanoparticle composition may optionally include one or more coatings. For example, the nanoparticle composition may be formulated in capsules, films, or tablets with a coating. Capsules, films, or tablets of the compositions described herein may have any useful size, tensile strength, hardness, or density.

The nanoparticle composition of the present disclosure can be prepared in various forms suitable for various routes of administration, such as liquid dosage forms (e.g., emulsions, microemulsions, nanoemulsions, solutions, suspensions, syrups, and elixirs), injectable dosage forms, solid dosage forms (e.g., capsules, tablets, pills, powders, and granules), dosage forms for topical (including buccal and sublingual), transdermal, and/or percutaneous administration (e.g., creams, ointments, pastes, creams, lotions, gels, powders, solutions, sprays, inhalants, and patches), dosage forms for vaginal administration (e.g., vaginal suppositories, tampons, creams, gels, pastes, foams, and sprays), dosage forms for implant administration (e.g., solids, semi-solids, gels), suspensions, powders, and other dosage forms.

### Pharmaceutical Compositions

The nanoparticle composition may be formulated in whole or in part as a pharmaceutical composition. The pharmaceutical composition may comprise one or more nanoparticle compositions.

For example, a pharmaceutical composition may comprise one or more nanoparticle compositions, including one or more different therapeutic and/or prophylactic agents. The pharmaceutical composition may further comprise one or more pharmaceutically acceptable excipients, such as those described herein. General guidance for formulating and manufacturing pharmaceutical compositions and agents can be found in Remington's The Science and Practice of Pharmacy, 21st Edition. Conventional excipients and auxiliary ingredients may be used in any pharmaceutical composition, unless any conventional excipient or auxiliary ingredient is incompatible with one or more components of the nanoparticle composition. The content of excipients in the pharmaceutical composition according to the present disclosure can be determined as needed by those skilled in the art.

The relative amounts of the one or more nanoparticle compositions, the one or more pharmaceutically acceptable excipients, and/or any additional ingredients in the pharmaceutical composition according to the present disclosure will vary depending on the properties, the size of the nanoparticle, and/or the condition of the subject being treated, and further depending on the route of administration of the composition. By way of example, a pharmaceutical composition may comprise from 0.1% to 100% (wt/wt) of one or more nanoparticle compositions.

In certain embodiments, the nanoparticle compositions and/or pharmaceutical compositions of the present disclosure are refrigerated or frozen for storage and/or transport (e.g., stored at a temperature of 4°C or lower, such as a temperature between about -150°C and about 0°C, or between about -80°C and about -20°C, e.g., about - 5°C, -10°C, -15°C, -20°C, -25°C, -30°C, -40°C, -50°C, -60°C, -70°C, -80°C, -90°C, -130°C, or -150°C).

For example, the nanoparticle compositions and/or pharmaceutical compositions disclosed herein may be stable for at least about 1 week, at least 2 weeks, at least 3 weeks, at least 4 weeks, at least 5 weeks, at least 6 weeks, at least 2 months, at least 4 months, at least 6 months, at least 8 months, at least 10 months, at least 12 months, at least 14 months, at least 16 months, at least 18 months, at least 20 months, at least 22 months, or at least 24 months, e.g., at a temperature of 4°C or lower (e.g., between about 4°C and -20°C). In one embodiment, the composition is stable for at least 4 weeks at 4°C. In certain embodiments, the pharmaceutical composition of the present disclosure comprises the nanoparticle composition disclosed herein and a pharmaceutically acceptable excipient selected from one or more of Tris, acetate (e.g., sodium acetate), citrate (e.g., sodium citrate), saline, PBS, and sucrose. In certain embodiments, the pharmaceutical composition of the present disclosure has a pH between about 7 and 8 (e.g., 6.8, 6.9, 7.0, 7.1, 7.2, 7.3, 7.4, 7.5, 7.6, 7.7, 7.8, 7.9, or 8.0, or between 7.5) and 8, or between 7 and 7.8.

In the context of the present disclosure, "stability" and "stable" refer to the resistance of the nanoparticle compositions and/or pharmaceutical compositions disclosed herein to chemical or physical changes (e.g., degradation, particle size change, aggregation, change in encapsulation, etc.) under given conditions of manufacture, preparation, transport, storage, and/or use (e.g., upon application of stresses such as shear force, freeze/thaw stress, etc.).

Nanoparticle compositions and/or pharmaceutical compositions comprising one or more nanoparticle compositions may be administered to any patient or subject, including those who may benefit from the therapeutic effect provided by delivery of a therapeutic and/or prophylactic agent to one or more specific cells, tissues, organs, or systems, or groups thereof.

While the term "composition" relates primarily to compositions suitable for administration to humans, those skilled in the art will understand that such compositions are generally suitable for administration to any other mammals.

Modifications to compositions suitable for administration to humans to make them suitable for administration to various animals are well known, and the ordinary skilled veterinary pharmacologist could design and/or perform such modifications with only ordinary (if any) experimentation. Subjects to whom the compositions are to be administered are contemplated to include, but are not limited to, humans, other primates, and other mammals, including commercially relevant mammals such as cattle, pigs, horses, sheep, cats, dogs, mice, and/or rats.

Pharmaceutical compositions comprising one or more nanoparticle compositions can be prepared by any method known in the pharmacological arts or developed hereafter. Generally, such preparation methods include combining the active ingredient with excipients and/or one or more other auxiliary ingredients, and then, if necessary or desirable, dividing, shaping, and/or packaging the product into the desired single or multiple forms or multiple dosage unit(s).

Pharmaceutical compositions according to the present disclosure may be prepared, packaged, and/or sold in bulk, as a single unit dose, and/or as multiple single unit doses. As used herein, a "unit dose" is a discrete amount of the pharmaceutical composition containing a predetermined amount of the active ingredient (e.g., nanoparticle composition). The amount of the active ingredient is generally equal to the dose of the active ingredient to be administered to a subject and/or a convenient fraction of that dose, such as one-half or one-third of that dose.

Pharmaceutical compositions may be prepared in a variety of forms suitable for various routes and methods of administration. For example, pharmaceutical compositions may be prepared as liquid dosage forms (e.g., emulsions, microemulsions, nanoemulsions, solutions, suspensions, syrups, and elixirs), injectable dosage forms, solid dosage forms (e.g., capsules, tablets, pills, powders, and granules), dosage forms for topical (including buccal and sublingual), transdermal, and/or percutaneous administration (e.g., creams, ointments, pastes, creams, lotions, gels, powders, solutions, sprays, inhalants, and patches), dosage forms for vaginal administration (e.g., vaginal suppositories, tampons, creams, gels, pastes, foams, and sprays), dosage forms for implant administration (e.g., solids, semi-solids, gels), suspensions, powders, and other dosage forms.

Liquid dosage forms for oral and parenteral administration include, but are not limited to, pharmaceutically acceptable emulsions, microemulsions, nanoemulsions, solutions, suspensions, syrups, and/or elixirs. In addition to the active ingredient, liquid dosage forms may comprise inert diluents commonly used in the art, such as water or other solvents, solubilizing agents, and emulsifiers, such as ethanol, isopropyl alcohol, ethyl carbonate, ethyl acetate, benzyl alcohol, benzyl benzoate, propylene glycol, 1,3-butanediol, dimethylformamide, oils (in particular cottonseed oil, peanut oil, corn oil, germ oil, olive oil, castor oil, and sesame oil), glycerol, tetrahydrofurfuryl alcohol, polyethylene glycol, and fatty acid esters of sorbitan, and mixtures thereof. In addition to inert diluents, oral compositions may include additional therapeutic and/or prophylactic agents, additional agents such as wetting agents, emulsifiers and suspending agents, sweeteners, flavorings, and/or aromas. In certain embodiments for parenteral administration, the composition is mixed with a solubilizing agent such as an alcohol, oil, modified oil, glycol, polysorbate, cyclodextrin, polymer, and/or combinations thereof.

Injectable preparations, such as sterile injectable aqueous or oily suspensions, may be formulated according to known techniques using suitable dispersing, wetting, and/or suspending agents. Sterile injectable preparations may be sterile injectable solutions, suspensions, and/or emulsions in non-toxic parenterally acceptable diluents and/or solvents. Acceptable carriers and solvents that may be used include water, Ringer's solution, and isotonic sodium chloride solution. Sterile oils may be used as solvents or suspending media. For this purpose, any mild non-volatile oil may be used, including synthetic mono- or diglycerides. Fatty acids such as oleic acid may be used in the preparation of injectables. Injectable formulations may be sterilized, for example, by filtration through a bacteria-retaining filter, and/or by incorporating sterilizing agents in the form of sterile solid compositions, which may be dissolved or dispersed in sterile water or other sterile injectable media prior to use.

Compositions for rectal or vaginal administration are generally suppositories, which may be prepared by mixing the composition with suitable non-irritating excipients that are solid at ambient temperature but liquid at body temperature and therefore melt in the rectal or vaginal cavity and release the active ingredient.

The present disclosure also contemplates the use of transdermal patches. Such dosage forms can be prepared, for example, by dissolving and/or dispersing the compound in an appropriate medium. Alternatively or additionally, the rate can be controlled by providing a rate-controlling membrane and/or by dispersing the compound in a polymer matrix and/or gel.

Suitable devices for delivering the intradermal pharmaceutical compositions described herein include any type of liquid or solid injection device, such as conventional syringes, fine-needle syringes, short-needle syringes, liquid jet injectors, compressed gas-accelerated powder injectors, ballistic powder delivery devices, and the like.

Pharmaceutical compositions may be prepared, packaged, and/or sold in formulations suitable for administration via the buccal cavity or pulmonary route. Such formulations may comprise dry particles comprising the active ingredient. Such compositions are conveniently in the form of dry powders for administration using devices comprising a dry powder reservoir and/or using self-propelled solvent/powder dispensing containers. Such formulations may also be prepared, packaged, and/or sold as aqueous and/or dilute alcoholic solutions and/or suspensions (optionally sterile, comprising the active ingredient) and conveniently administered using any nebulizing and/or atomizing device. Such formulations may further comprise one or more additional ingredients, including but not limited to flavoring agents such as saccharin sodium, volatile oils, buffers, surfactants, and/or preservatives such as methyl hydroxybenzoate.

Formulations described herein suitable for pulmonary delivery may also be used for intranasal delivery of pharmaceutical compositions. Such formulations are administered intranasally, i.e., by rapid inhalation through the nasal passage from a powder container held close to the nose. Pharmaceutical compositions may be prepared, packaged, and/or sold in formulations suitable for oral administration. Such formulations may be, for example, in the form of tablets and/or lozenges prepared using conventional methods.

### Methods and Uses

The present disclosure provides methods for producing a polypeptide of interest in mammalian cells. Methods for producing a polypeptide involve contacting cells with a nanoparticle composition comprising an mRNA encoding the polypeptide of interest. When cells are contacted with the nanoparticle composition, the mRNA can be taken up and translated in the cells to produce the polypeptide of interest.

Generally, the step of contacting mammalian cells with a nanoparticle composition comprising an mRNA encoding a polypeptide of interest can be performed in vivo, ex vivo, in culture, or in vitro. The amount of the nanoparticle composition and/or the mRNA therein contacted with cells may depend on the type of cells or tissue being contacted, the mode of administration, the physicochemical characteristics (e.g., size, charge, and chemical composition) of the nanoparticle composition and mRNA, and other factors. Metrics of efficiency may include polypeptide translation (indicated by polypeptide expression), levels of mRNA degradation, and indicators of immune response.

The step of contacting cells with a nanoparticle composition comprising mRNA may involve or cause transfection. Phospholipids contained in the lipid component of the nanoparticle composition may, for example, promote transfection and/or increase transfection efficiency by interacting with and/or fusing with cell membranes or intracellular membranes. Transfection may allow translation of the mRNA inside the cell.

In some embodiments, the nanoparticle compositions described herein may be used for therapy. For example, the mRNA contained in the nanoparticle composition may encode a therapeutic polypeptide (e.g., in a translatable region) and produce the therapeutic polypeptide after contacting and/or entering (e.g., transfecting) a cell. In other embodiments, the mRNA contained in the nanoparticle composition may encode a polypeptide that improves or increases immunity in a subject.

In certain embodiments, the mRNA contained in the nanoparticle composition may encode a recombinant polypeptide that may replace one or more polypeptides substantially absent in the cells contacted with the nanoparticle composition. Alternatively, the recombinant polypeptide produced by mRNA translation may antagonize the activity of an endogenous protein in, on the surface of, or secreted from the cell. In another alternative, the recombinant polypeptide produced by mRNA translation may indirectly or directly antagonize the activity of a biological part in, on the surface of, or secreted from the cell. Antagonized biological parts may include, but are not limited to, lipids (e.g., cholesterol), lipoproteins (e.g., low-density lipoprotein), nucleic acids, carbohydrates, and small molecule toxins.

### Methods for Delivering Therapeutic Agents to Cells and Organs

The present disclosure provides methods of delivering a therapeutic and/or prophylactic agent to mammalian cells or organs. Delivering a therapeutic and/or prophylactic agent to cells involves administering to a subject a nanoparticle composition comprising the therapeutic and/or prophylactic agent, wherein administration of the composition involves contacting the cells with the composition. For example, a protein, cytotoxin, radioactive ion, chemotherapeutic agent, or nucleic acid (e.g., RNA, such as mRNA) may be delivered to cells or an organ. When the therapeutic and/or prophylactic agent is an mRNA, upon contact of the cell with the nanoparticle composition, the translatable mRNA may be translated in the cell to produce the polypeptide of interest. However, essentially non-translatable mRNA may also be delivered to cells.

In some embodiments, the nanoparticle composition can be targeted to specific types of cells (e.g., cells of a specific organ or system thereof). For example, a nanoparticle composition comprising a therapeutic and/or prophylactic agent of interest may be specifically delivered to a mammalian liver, kidney, spleen, femur, or lung with substantially no delivery to other types of cells. Specific delivery to a specific type of cell, organ, or system or tissue thereof means that a higher proportion of the nanoparticle composition comprising the therapeutic and/or prophylactic agent is delivered to the destination of interest relative to other destinations. In some embodiments, the tissue of interest is selected from liver, kidney, lung, spleen, femur, muscle, tumor tissue (e.g., via intratumoral injection).

As another example of targeting or specific delivery, mRNA encoding a ligand that binds a protein on the cell surface (e.g., an antibody or functional fragment thereof, scaffold protein, or peptide) or a receptor may be included in the nanoparticle composition. In some embodiments, the ligand may be a surface-bound antibody, which may allow modulation of cell targeting specificity. These methods may improve the affinity and specificity of targeting interactions. Ligands can be selected, for example, by those skilled in the biological arts based on the desired localization or function of the cell. Target cells may include, but are not limited to, hepatocytes, epithelial cells, hematopoietic cells, endothelial cells, lung cells, bone cells, stem cells, mesenchymal cells, nerve cells, cardiomyocytes, adipocytes, vascular smooth muscle cells, skeletal muscle cells, beta cells, pituitary cells, synovial lining cells, ovarian cells, testicular cells, fibroblasts, B cells, T cells, reticulocytes, leukocytes, granulocytes, and tumor cells.

### Methods for Treating Diseases and Disorders

The nanoparticle compositions may be used to treat a disease or disorder. In particular, such compositions may be used to treat diseases or disorders characterized by absence or abnormal activity of a protein or polypeptide. For example, a nanoparticle composition comprising an mRNA encoding a missing or abnormal polypeptide may be administered or delivered to cells. Subsequent translation of the mRNA can produce the polypeptide, thereby reducing or eliminating problems caused by the absence of activity or abnormal activity due to the polypeptide. The methods and compositions can be used to treat acute diseases or conditions, such as sepsis, stroke, and myocardial infarction. Therapeutic and/or prophylactic agents contained in nanoparticle compositions may also be able to alter transcription rates of a given species, thereby affecting gene expression. Diseases and/or disorders characterized by dysfunction or abnormality of a protein or polypeptide for which compositions may be administered include, but are not limited to, rare diseases, infectious diseases (as vaccines and therapeutics), cancers and proliferative diseases, genetic diseases (e.g., cystic fibrosis), autoimmune diseases, diabetes, neurodegenerative diseases, cardiovascular and renovascular diseases, and metabolic diseases.

The present disclosure provides methods involving administering a nanoparticle composition comprising one or more therapeutic and/or prophylactic agents and pharmaceutical compositions comprising the same. The terms "therapeutic" and "prophylactic" may be used interchangeably herein. Therapeutic compositions or an imaging, diagnostic, or prophylactic compositions thereof may be administered to a subject in any reasonable amount and via any administration route effective for prevention, treatment, diagnosis, or imaging a disease, disorder, and/or condition. The specific amount administered to a subject may vary based on the species, age, and general condition of the subject, the specific components, and the mode of administration. Compositions according to the present disclosure may be formulated in dosage unit form for ease of administration and uniformity of dosage. However, it should be understood that the specific dosage of the compositions of the present disclosure will be determined by the attending physician within the scope of sound medical judgment.

Nanoparticle compositions comprising one or more therapeutic and/or prophylactic agents may be administered by any route. In some embodiments, the composition, including a prophylactic, diagnostic, or imaging composition comprising one or more nanoparticle compositions as described herein, is administered via one or more of a variety of routes, including oral, intravenous, intramuscular, intraarterial, intramedullary, intrathecal, subcutaneous, intraventricular, transdermal or intradermal, interdermal, rectal, intravaginal, intraperitoneal, intraocular, subretinal, intravitreal, mucosal, intranasal, oral, enteral, intratumoral, sublingual, intranasal; by intratracheal instillation, bronchial instillation, and/or inhalation, as oral sprays and/or powders, nasal sprays and/or aerosols, and/or via a portal vein catheter. Local administration is preferred, e.g., intramuscular, subcutaneous, transdermal or intradermal, interdermal, intraperitoneal, intraocular, subretinal, intravitreal, mucosal, intranasal, oral, intratumoral, intranasal. Suitable routes of administration will depend on a variety of factors, including the nature of the nanoparticle composition, the therapeutic and/or prophylactic agent, the patient's condition, etc.

In certain embodiments, compositions according to the present disclosure may be administered at levels sufficient to deliver a dose of: about 0.0001 mg/kg to about 10 mg/kg, about 0.001 mg/kg to about 10 mg/kg, about 0.005 mg/kg to about 10 mg/kg, about 0.01 mg/kg to about 10 mg/kg, about 0.05 mg/kg to about 10 mg/kg, about 0.1 mg/kg to about 10 mg/kg, about 1 mg/kg to about 10 mg/kg, about 2 mg/kg to about 10 mg/kg, about 5 mg/kg to about 10 mg/kg, about 0.0001 mg/kg to about 5 mg/kg, about 0.001 mg/kg to about 5 mg/kg, about 0.005 mg/kg to about 5 mg/kg, about 0.01 mg/kg to about 5 mg/kg, about 0.05 mg/kg to about 5 mg/kg, about 0.1 mg/kg to about 5 mg/kg, about 1 mg/kg to about 5 mg/kg, about 2 mg/kg to about 5 mg/kg, about 0.0001 mg/kg to about 2.5 mg/kg, about 0.001 mg/kg to about 2.5 mg/kg, about 0.005 mg/kg to about 2.5 mg/kg, about 0.01 mg/kg to about 2.5 mg/kg, about 0.05 mg/kg to about 2.5 mg/kg, about 0.1 mg/kg to about 2.5 mg/kg, about 1 mg/kg to about 2.5 mg/kg, about 2 mg/kg to about 2.5 mg/kg, about 0.0001 mg/kg to about 1 mg/kg, about 0.001 mg/kg to about 1 mg/kg, about 0.005 mg/kg to about 1 mg/kg, about 0.01 mg/kg to about 1 mg/kg, about 0.05 mg/kg to about 1 mg/kg, about 0.1 mg/kg to about 1 mg/kg, about 0.0001 mg/kg to about 0.25 mg/kg, about 0.001 mg/kg to about 0.25 mg/kg, about 0.005 mg/kg to about 0.25 mg/kg, about 0.01 mg/kg to about 0.25 mg/kg, about 0.05 mg/kg to about 0.25 mg/kg, or about 0.1 mg/kg to about 0.25 mg/kg of the therapeutic and/or prophylactic agent (e.g., mRNA).

The dose may be administered once or multiple times per day at the same or different amounts to achieve the desired level of mRNA expression and/or therapeutic, diagnostic, prophylactic, or imaging effect. The desired dose may be delivered, for example, three times daily, twice daily, once daily, every other day, every three days, weekly, every two weeks, every three weeks, or every four weeks. In certain embodiments, the desired dose may be delivered using multiple administrations (e.g., two, three, four, five, six, seven, eight, nine, ten, eleven, twelve, thirteen, fourteen, or more administrations).

Nanoparticle compositions comprising one or more therapeutic and/or prophylactic agents may be used in combination with one or more other therapeutic agents, prophylactic agents, diagnostic agents, or imaging agents. Each agent will be administered at the dose and/or schedule determined for that agent. The therapeutic, prophylactic, diagnostic, or imaging active agents for use in combination may be administered together in a single composition or separately in different compositions.

Those skilled in the art will recognize or be able to ascertain using no more than routine experimentation many equivalents to the specific embodiments described herein. The scope of the present disclosure is not intended to be limited to the above description but is as set forth in the appended claims.

In the claims, articles such as "a", "an", and "the" may mean one or more than one, unless indicated to the contrary or apparent from context.

The term "comprising" is intended to be open-ended and allows but does not require the inclusion of additional elements or steps. When the term "comprising" is used herein, the terms "consisting essentially of ..." and "consisting of ..." are thus also encompassed and disclosed. Furthermore, it should be understood that the order of steps or performing certain actions is not important, as long as the disclosure remains operable. Two or more steps or actions may be performed simultaneously.

Compounds of the present disclosure may be prepared in a variety of ways using commercially available starting materials, compounds known in the literature, or intermediates readily prepared, by employing standard synthetic methods and procedures known to those skilled in the art or to be learned. The synthesis of compounds of the present disclosure will be apparent to those skilled in the art in view of the teachings herein. Standard synthetic methods and procedures for preparing organic molecules as well as functional group transformations and manipulations can be found in the relevant scientific literature or standard textbooks in the field, such as Smith, M. B., March, J., March's Advanced Organic Chemistry: Reactions, Mechanisms, and Structure, 5th edition, John Wiley & Sons: New York, 2001. The synthetic methods described in the Examples herein are intended to illustrate, but not limit, the general procedures for preparing the compounds of the present disclosure. Those of ordinary skill in the art will note that the order of certain steps in the reaction sequences and synthetic schemes described herein may be varied, for example the introduction and removal of protecting groups. In the reaction schemes described herein, a variety of stereoisomers may be produced. Those of ordinary skill in the art will recognize that reactions may be optimized to preferentially produce one isomer, or new schemes may be devised to produce a single isomer. If mixtures are produced, techniques such as preparative thin-layer chromatography, preparative HPLC, preparative chiral HPLC, or preparative SFC may be used to separate the isomers.

In one or more embodiments, the compound of Formula (I) described herein is Compounds 1-7 as shown below:

| | |
|---|---|
| **1** | 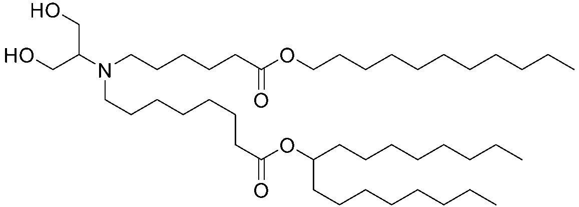 |
| **2** | 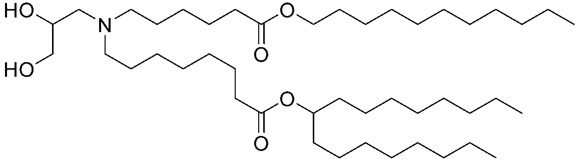 |
| **3** | 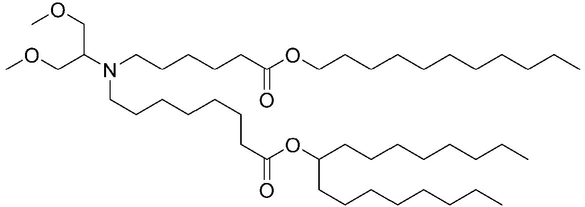 |
| **4** | 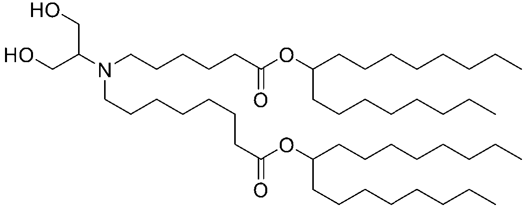 |
| **5** | 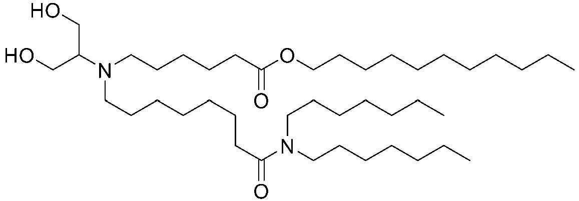 |
| **6** | 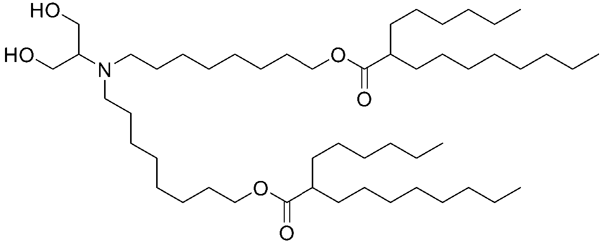 |
| **7** | 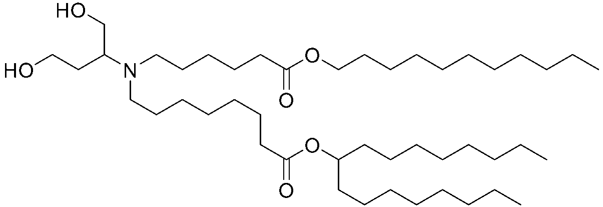 |

### EXAMPLES

### Example 1: Preparation of Compounds

### Experimental Instruments

| **Instrument** | **Model/Source** |
|---|---|
| Nuclear Magnetic Resonance Spectrometer | Varian-600MHz |
| High-Resolution Mass Spectrometer | Thermo LTQ Orbitrap XL |

| Experimental Reagents | |
|---|---|
| **Reagent** | **Source** |
| 8-Bromooctanoic acid | Energy Chemical |
| Heptadecan-9-ol | Energy Chemical |
| 1-(3-Dimethylaminopropyl)-3-ethylcarbodiimide hydrochloride | Bide Pharmatech |
| 4-Dimethylaminopyridine | Energy Chemical |
| Dichloromethane / Petroleum ether / Ethyl acetate / Methanol / Ethanol / Acetonitrile | Energy Chemical |
| 6-Bromohexanoic acid | Energy Chemical |
| Undecanol | Energy Chemical |
| 2-Aminopropane-1,3-diol | Energy Chemical |
| 3-Aminopropane-1,2-diol | Energy Chemical |
| 2-Amino-1,3-dimethoxypropane | Energy Chemical |
| 2-Hexyldecanoic acid | Bide Pharmatech |
| 8-Bromooctan-1-ol | Bide Pharmatech |
| 2-Aminobutane-1,4-diol | Energy Chemical |
| Potassium carbonate | Energy Chemical |
| Tetrabutylammonium iodide | Energy Chemical |
| Silica gel | Qingdao Haiyang Chemical Co., Ltd. |

### Synthetic Procedure for Compound 1:

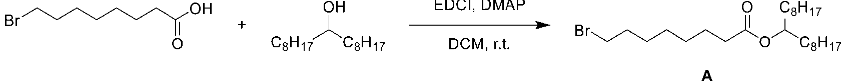

### Synthesis of Intermediate A (Heptadecan-9-yl 8-bromooctanoate)

A dry 100 mL round-bottom flask equipped with a stir bar was charged with 8-bromooctanoic acid (5.5 mmol, 1.22 g), heptadecan-9-ol (5.0 mmol, 1.28 g), 1-(3-dimethylaminopropyl)-3-ethylcarbodiimide hydrochloride (5.5 mmol, 1.05 g), 4-dimethylaminopyridine (0.5 mmol, 61 mg), and dichloromethane (15 mL). The mixture was stirred at room temperature for 24 hours. Upon completion of the reaction, the organic solvent was removed using a rotary evaporator to afford the crude product, which was then purified by column chromatography (petroleum ether/ethyl acetate = 20:1) to yield Intermediate A as a colorless liquid (2.12 g, yield 92%).

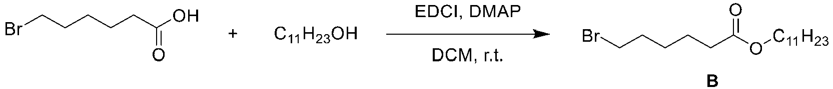

### Synthesis of Intermediate B (Undecyl 6-bromohexanoate)

A dry 100 mL round-bottom flask equipped with a stir bar was charged with 6-bromohexanoic acid (5.5 mmol, 1.07 g), undecanol (5.0 mmol, 0.86 g), 1-(3-dimethylaminopropyl)-3-ethylcarbodiimide hydrochloride (5.5 mmol, 1.05 g), 4-dimethylaminopyridine (0.5 mmol, 61 mg), and dichloromethane (15 mL). The mixture was stirred at room temperature for 24 hours. Upon completion of the reaction, the organic solvent was removed using a rotary evaporator to afford the crude product, which was then purified by column chromatography (petroleum ether/ethyl acetate = 20:1) to yield Intermediate B as a colorless liquid (1.46 g, yield 84%).

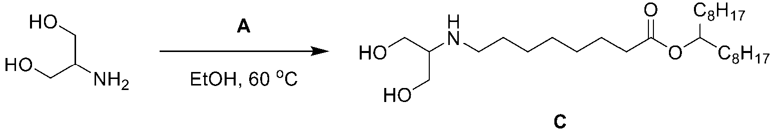

### Synthesis of Intermediate C (Heptadecan-9-yl 8-[(1,3-dihydroxypropan-2-yl)amino]octanoate)

A dry 100 mL round-bottom flask equipped with a stir bar was charged with heptadecan-9-yl 8-bromooctanoate (5.0 mmol, 2.3 g), 2-aminopropane-1,3-diol (50 mmol, 4.5 g), and anhydrous ethanol (30 mL). The mixture was stirred at 60 °C for 24 hours. Upon completion of the reaction, the organic solvent was removed using a rotary evaporator to afford the crude product, which was then purified by column chromatography (dichloromethane/methanol = 10:1) to yield Intermediate C as a colorless liquid (942 mg, yield 40%).

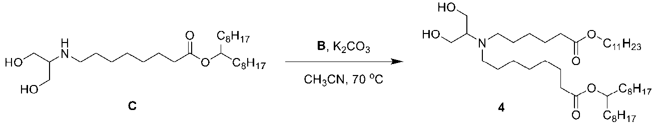

### Synthesis of Compound 1 (Heptadecan-9-yl 8-[1-hydroxy-2-(hydroxymethyl)-9-oxo-3-aza-10-oxahenicosan-3-yl]octanoate)

A dry 100 mL round-bottom flask equipped with a stir bar was charged with heptadecan-9-yl 8-[(1,3-dihydroxypropan-2-yl)amino]octanoate (1.5 mmol, 706 mg), undecyl 6-bromohexanoate (1.7 mmol, 486 mg), potassium carbonate (1.8 mmol, 248 mg), tetrabutylammonium iodide (0.075 mmol, 27 mg), and anhydrous acetonitrile (8 mL). The mixture was stirred under reflux at 70 °C for 24 hours. Upon completion of the reaction, the organic solvent was removed using a rotary evaporator to afford the crude product, which was then purified by column chromatography (dichloromethane/methanol = 20:1) to yield Compound 1 as a colorless liquid (389 mg, yield 35%). **¹H NMR** (600 MHz, Chloroform-d) *δ* 4.86 (p, *J =* 6.3 Hz, 1H), 4.05 (t, *J =* 6.8 Hz, 2H), 3.59 (d, *J =* 6.8 Hz, 4H), 2.96 (p, *J =* 6.9 Hz, 1H), 2.56 (q, *J =* 9.2, 8.6 Hz, 4H), 2.29 (dt, *J =* 13.2, 7.5 Hz, 4H), 1.66 - 1.55 (m, 6H), 1.53 - 1.42 (m, 8H), 1.33 - 1.21 (m, 50H), 0.91 - 0.82 (m, 9H). **HRMS** (ESI) m/z (M+H)⁺ calculated for C45H90NO6: 740.6768, observed: 740.6775.

### Synthetic Procedure for Compound 2:

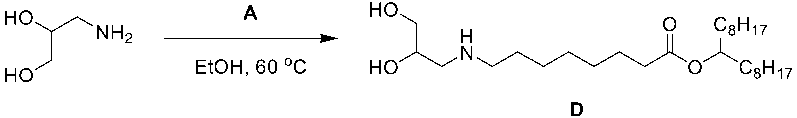

### Synthesis of Intermediate D (Heptadecan-9-yl 8-[(2,3-dihydroxypropyl)amino]octanoate)

A dry 100 mL round-bottom flask equipped with a stir bar was charged with heptadecan-9-yl 8-bromooctanoate (5.0 mmol, 2.3 g), 3-aminopropane-1,2-diol (50 mmol, 4.5 g), and anhydrous ethanol (30 mL). The mixture was stirred at 60 °C for 24 hours. Upon completion of the reaction, the organic solvent was removed using a rotary evaporator to afford the crude product, which was then purified by column chromatography (dichloromethane/methanol = 10:1) to yield Intermediate D as a colorless liquid (786 mg, yield 33%).

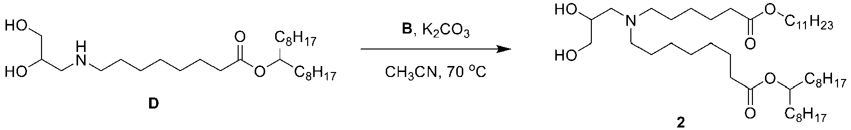

### Synthesis of Compound 2 (Heptadecan-9-yl 8-(1,2-dihydroxy-10-oxo-4-aza-11-oxadocosan-4-yl)octanoate)

A dry 100 mL round-bottom flask equipped with a stir bar was charged with Intermediate D (1.5 mmol, 706 mg), undecyl 6-bromohexanoate (1.7 mmol, 486 mg), potassium carbonate (1.8 mmol, 248 mg), tetrabutylammonium iodide (0.075 mmol, 27 mg), and anhydrous acetonitrile (8 mL). The mixture was stirred under reflux at 70 °C for 24 hours. Upon completion of the reaction, the organic solvent was removed using a rotary evaporator to afford the crude product, which was then purified by column chromatography (dichloromethane/methanol = 20:1) to yield Compound 2 as a colorless liquid (478 mg, yield 43%). **¹H NMR** (600 MHz, Chloroform-*d*) *δ* 4.86 (p, *J =* 6.5 Hz, 1H), 4.05 (t, *J =* 6.8 Hz, 2H), 3.92 - 3.85 (m, 1H), 3.75 (dd, *J =* 11.5*,* 4.0 Hz, 1H), 3.52 (dd, *J =* 11.4, 4.2 Hz, 1H), 2.80 - 2.73 (m, 1H), 2.73 - 2.65 (m, 2H), 2.64 - 2.55 (m, 3H), 2.29 (dt, *J =* 18.0, 7.4 Hz, 4H), 1.68 - 1.47 (m, 14H), 1.36 - 1.17 (m, 50H), 0.87 (t, *J =* 7.0 Hz, 9H).

### Synthetic Procedure for Compound 3:

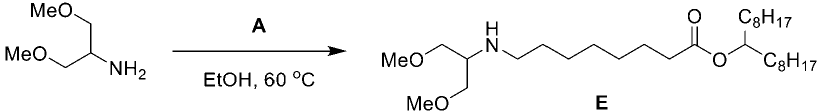

### Synthesis of Intermediate E (Heptadecan-9-yl 8-(2,6-dioxaheptan-4-ylamino)octanoate)

A dry 100 mL round-bottom flask equipped with a stir bar was charged with heptadecan-9-yl 8-bromooctanoate (5.0 mmol, 2.3 g), 2-amino-1,3-dimethoxypropane (50 mmol, 5.95 g), and anhydrous ethanol (30 mL). The mixture was stirred at 60 °C for 24 hours. Upon completion of the reaction, the organic solvent was removed using a rotary evaporator to afford the crude product, which was then purified by column chromatography (dichloromethane/methanol = 10:1) to yield Intermediate E as a colorless liquid (1.45 g, yield 58%).

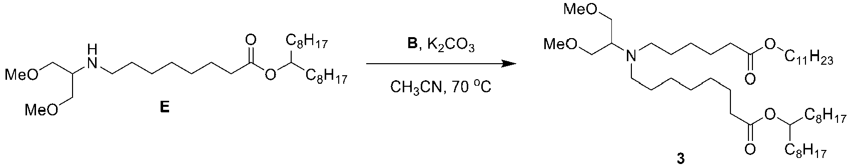

### Synthesis of Compound 3 (Heptadecan-9-yl 8-[4-(methoxymethyl)-11-oxo-5-aza-2,12-dioxatricosan-5-yl]octanoate)

A dry 100 mL round-bottom flask equipped with a stir bar was charged with Intermediate E (1.5 mmol, 749 mg), undecyl 6-bromohexanoate (1.7 mmol, 486 mg), potassium carbonate (1.8 mmol, 248 mg), tetrabutylammonium iodide (0.075 mmol, 27 mg), and anhydrous acetonitrile (8 mL). The mixture was stirred under reflux at 70°C for 24 hours. Upon completion of the reaction, the organic solvent was removed using a rotary evaporator to afford the crude product, which was then purified by column chromatography (dichloromethane/methanol = 20:1) to yield Compound 3 as a colorless liquid (622 mg, yield 54%). **¹H NMR** (600 MHz, Chloroform-d) *δ* 4.84 (p, *J* = 6.3 Hz, 1H), 4.02 (t, *J* = 6.8 Hz, 2H), 3.44 (dd, *J* = 9.7, 5.9 Hz, 2H), 3.35 (dd, *J* = 9.7, 5.9 Hz, 2H), 3.29 (s, 6H), 2.96 (p, *J* = 5.9 Hz, 1H), 2.47 (dt, *J* = 9.7, 7.2 Hz, 4H), 2.25 (dt, *J* = 9.6, 7.5 Hz, 4H), 1.64 - 1.52 (m, 6H), 1.47 (q, *J* = 7.0, 6.2 Hz, 4H), 1.43 - 1.33 (m, 4H), 1.30 - 1.17 (m, 48H), 0.89 - 0.82 (m, 9H). **HRMS** (ESI) m/z (M+H)⁺ calculated for C47H94NO6: 768.7081, observed: 768.6803.

### Synthetic Procedure for Compound 4:

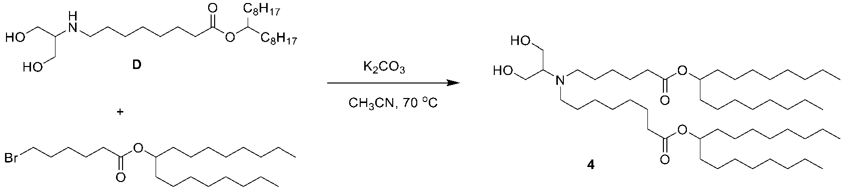

### Synthesis of Compound 4 (Heptadecan-9-yl 8-[1-hydroxy-2-(hydroxymethyl)-11-octyl-9-oxo-3-aza-10-oxanonadecan-3-yl]octanoate)

A dry 100 mL round-bottom flask equipped with a stir bar was charged with Intermediate D (0.2 mmol, 94.2 mg), heptadecan-9-yl 6-bromohexanoate (0.3 mmol, 129.6 mg), potassium carbonate (0.3 mmol, 41.4 mg), tetrabutylammonium iodide (0.01 mmol, 3.7 mg), and anhydrous acetonitrile (4 mL). The mixture was stirred under reflux at 70 °C for 24 hours. Upon completion of the reaction, the organic solvent was removed using a rotary evaporator to afford the crude product, which was then purified by column chromatography (dichloromethane/methanol = 20:1) to yield Compound 4 as a colorless liquid (61 mg, yield 37%). **¹H NMR** (600 MHz, Chloroform-*d*) *δ* 4.83 (p, *J* = 6.3 Hz, 2H), 3.58 (d, *J* = 6.8 Hz, 4H), 2.98 (p, *J* = 6.8 Hz, 1H), 2.60 - 2.54 (m, 4H), 2.25 (q, *J* = 7.2 Hz, 4H), 1.59 (dt, *J* = 14.4, 7.3 Hz, 4H), 1.51 - 1.41 (m, 12H), 1.31 - 1.18 (m, 58H), 0.85 (t, *J* = 7.0 Hz, 12H).

### Synthetic Procedure for Compound 5:

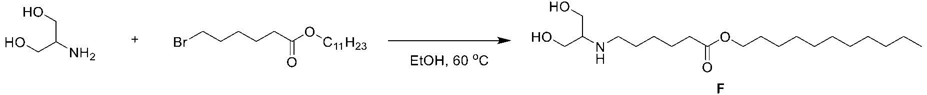

### Synthesis of Intermediate F (Undecyl 6-[(1,3-dihydroxypropan-2-yl)amino]hexanoate)

A dry 100 mL round-bottom flask equipped with a stir bar was charged with undecyl 6-bromohexanoate (5.0 mmol, 2.3 g), 2-aminopropane-1,3-diol (50 mmol, 4.55 g), and anhydrous ethanol (30 mL). The mixture was stirred at 60 °C for 24 hours. Upon completion of the reaction, the organic solvent was removed using a rotary evaporator to afford the crude product, which was then purified by column chromatography (dichloromethane/methanol = 10:1) to yield Intermediate F as a colorless liquid (826 mg, yield 46%).

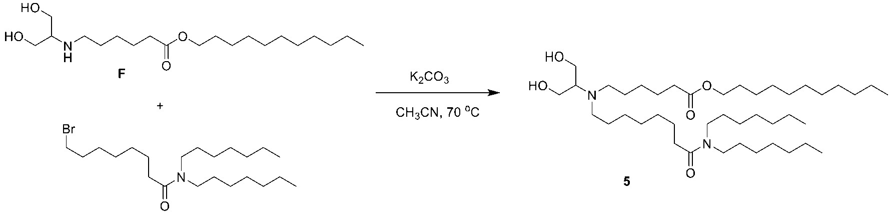

### Synthesis of Compound 5 (Undecyl 6-[12-heptyl-1-hydroxy-2-(hydroxymethyl)-11-oxo-3,12-diazanonadecan-3-yl]hexanoate)

A dry 100 mL round-bottom flask equipped with a stir bar was charged with Intermediate F (0.2 mmol, 71.8 mg), 8-bromo-N,N-diheptyloctanamide (0.3 mmol, 1025.1 mg), potassium carbonate (0.3 mmol, 41.4 mg), tetrabutylammonium iodide (0.01 mmol, 3.7 mg), and anhydrous acetonitrile (3 mL). The mixture was stirred under reflux at 70 °C for 24 hours. Upon completion of the reaction, the organic solvent was removed using a rotary evaporator to afford the crude product, which was then purified by column chromatography (dichloromethane/methanol = 20:1) to yield Compound 5 as a colorless liquid (61.2 mg, yield 44%). **¹H NMR** (600 MHz, Chloroform-d) *δ* 4.00 (t, *J* = 6.8 Hz, 2H), 3.66 (d, *J* = 6.5 Hz, 4H), 3.30 - 3.25 (m, 1H), 3.24 - 3.20 (m, 2H), 3.19 - 3.12 (m, 2H), 2.76 (t, *J* = 7.7 Hz, 4H), 2.25 (dt, *J* = 15.8, 7.5 Hz, 4H), 1.68 - 1.37 (m, 16H), 1.32 - 1.17 (m, 40H), 0.88 - 0.75 (m, 9H).

### Synthetic Procedure for Compound 6:

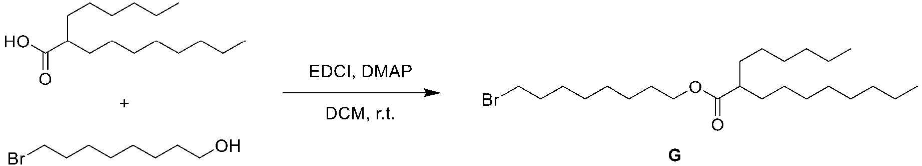

### Synthesis of Intermediate G (8-Bromooctyl 2-hexyldecanoate)

A dry 100 mL round-bottom flask equipped with a stir bar was charged with 2-hexyldecanoic acid (5.5 mmol, 1.41 g), 8-bromooctan-1-ol (5.0 mmol, 1.04 g), 1-(3-dimethylaminopropyl)-3-ethylcarbodiimide hydrochloride (5.5 mmol, 1.05 g), 4-dimethylaminopyridine (0.5 mmol, 61 mg), and dichloromethane (15 mL). The mixture was stirred at room temperature for 24 hours. Upon completion of the reaction, the organic solvent was removed using a rotary evaporator to afford the crude product, which was then purified by column chromatography (petroleum ether/ethyl acetate = 40:1) to yield Intermediate G as a colorless liquid (1.87 g, yield 84%).

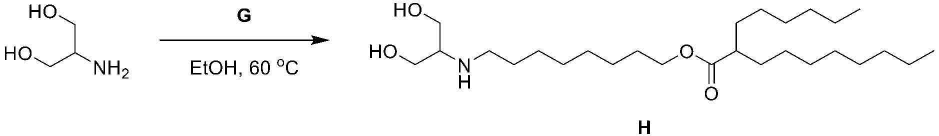

### Synthesis of Intermediate H (8-[(1,3-Dihydroxypropan-2-yl)amino]octyl 2-hexyldecanoate)

A dry 100 mL round-bottom flask equipped with a stir bar was charged with Intermediate G (3.0 mmol, 1.34 g), 2-aminopropane-1,3-diol (30 mmol, 3.73 g), and anhydrous ethanol (30 mL). The mixture was stirred at 60 °C for 24 hours. Upon completion of the reaction, the organic solvent was removed using a rotary evaporator to afford the crude product, which was then purified by column chromatography (dichloromethane/methanol = 10:1) to yield Intermediate H as a colorless liquid (576 mg, yield 42%).

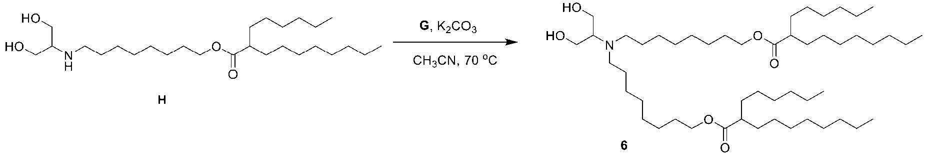

### Synthesis of Compound 6 (2-Hexyldecanoic acid 20-(1,3-dihydroxypropan-2-yl)-9-hexyl-10-oxo-20-aza-11-oxaoctacosan-28-yl ester)

A dry 100 mL round-bottom flask equipped with a stir bar was charged with Intermediate H (0.2 mmol, 91.4 mg), Intermediate G (0.3 mmol, 134 mg), potassium carbonate (0.3 mmol, 41.4 mg), tetrabutylammonium iodide (0.02 mmol, 7.4 mg), and anhydrous acetonitrile (3 mL). The mixture was stirred under reflux at 70 °C for 24 hours. Upon completion of the reaction, the organic solvent was removed using a rotary evaporator to afford the crude product, which was then purified by column chromatography (dichloromethane/methanol = 20:1) to yield Compound 6 as a colorless liquid (84 mg, yield 51%). **¹H NMR** (600 MHz, Chloroform-*d*) *δ* 4.04 (t, *J* = 6.7 Hz, 4H), 3.62 (d, *J* = 6.7 Hz, 4H), 3.03 (p, *J* = 6.7 Hz, 1H), 2.62 (t, *J* = 7.6 Hz, 4H), 2.29 (tt, *J* = 9.4, 5.2 Hz, 2H), 1.64 - 1.52 (m, 9H), 1.51 - 1.45 (m, 4H), 1.44 - 1.37 (m, 4H), 1.33 - 1.17 (m, 57H), 0.86 (t, *J* = 6.9 Hz, 12H).

### Synthetic Procedure for Compound 7:

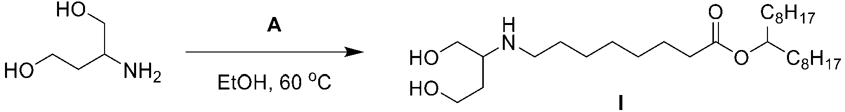

### Synthesis of Intermediate I (Heptadecan-9-yl 8-[(1,4-dihydroxybutan-2-yl)amino]octanoate)

A dry 100 mL round-bottom flask equipped with a stir bar was charged with Intermediate A (5.0 mmol, 2.3 g), 2-aminobutane-1,4-diol (50 mmol, 5.25 g), and anhydrous ethanol (30 mL). The mixture was stirred at 60 °C for 24 hours. Upon completion of the reaction, the organic solvent was removed using a rotary evaporator to afford the crude product, which was then purified by column chromatography (dichloromethane/methanol = 10:1) to yield Intermediate I as a colorless liquid (800 mg, yield 33%).

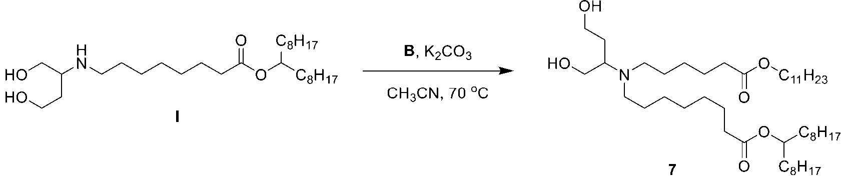

### Synthesis of Compound 7 (Heptadecan-9-yl 8-[1-hydroxy-3-(hydroxymethyl)-10-oxo-4-aza-11-oxadocosan-4-yl]octanoate)

A dry 100 mL round-bottom flask equipped with a stir bar was charged with Intermediate I (0.2 mmol, 97 mg), Intermediate B (0.3 mmol, 104.4 mg), potassium carbonate (0.3 mmol, 41.4 mg), tetrabutylammonium iodide (0.02 mmol, 7.4 mg), and anhydrous acetonitrile (3 mL). The mixture was stirred under reflux at 70 °C for 24 hours. Upon completion of the reaction, the organic solvent was removed using a rotary evaporator to afford the crude product, which was then purified by column chromatography (dichloromethane/methanol = 20:1) to yield Compound 7 as a colorless liquid (78.4 mg, yield 52%). **¹H NMR** (600 MHz, Chloroform-*d*) *δ* 4.85 (p, *J* = 6.3 Hz, 1H), 4.05 (t, *J* = 6.8 Hz, 2H), 3.75 - 3.67 (m, 2H), 3.58 - 3.46 (m, 2H), 3.05 - 2.92 (m, 1H), 2.49 (q, *J* = 8.7 Hz, 4H), 2.28 (dt, *J* = 14.8, 7.5 Hz, 4H), 1.66 - 1.57 (m, 6H), 1.54 - 1.42 (m, 6H), 1.34 - 1.19 (m, 54H), 0.93 - 0.79 (m, 9H).

### Example 2: Detection of Local Expression Activity of mRNA-LNP

### Experimental Materials

| **Instrument/Reagent Name** | **Manufacturer** | **Catalog No./Batch No.** |
|---|---|---|
| Compounds 1-10 | Self-synthesized | - |
| SM-102 | A.V.T. Pharmaceutical Co., Ltd. | SM220908 |
| PEG Lipid | A.V.T. Pharmaceutical Co., Ltd. | EDM210824 |
| Cholesterol | A.V.T. Pharmaceutical Co., Ltd. | C00373 |
| DSPC | A.V.T. Pharmaceutical Co., Ltd. | C20111 |
| Anhydrous Ethanol | Sinopharm Chemical Reagent Co., Ltd. | 10009218 |
| Luc mRNA | CATALYST Co., Ltd. | P22J012 |
| DEPC Water | Biosharp | 22321483 |
| PBS | Wuhan Servicebio Technology Co., Ltd. | G4202 |
| Microfluidic Instrument | ATSON BioTech | MPE-L2 |
| PDMS Chip | ATSON BioTech | SP.000011 |
| Isoflurane | RWD Life Science Co., Ltd. | 21060901 |
| 10%TritonX-100 | Beyotime Biotechnology | ST797 |
| 3M Sodium Acetate | Beyotime Biotechnology | ST351 |
| Ultrafiltration Tube | Millipore | UFC910096 |
| RNA Quantification Kit | ThermoFisher Scientific | R11490 |
| C57 Mice | Shanghai SLAC Laboratory Animal Co., Ltd. | - |
| Fine-Gauge Syringe | BD | |
| In Vivo Substrate | Promega | P1043 |
| Particle Size Analyzer | Malven | Nanozs |
| Small Animal In Vivo Imaging System | Perkin Elmer | IVIS Lumina K Series III |
| Spark Multimode Microplate Reader | TECAN | 30086376 |
| Ultracentrifuge | ThermoFisher Scientific | 75005289 |

### Experimental Methods

### 1. Preparation of LNP

Using Compounds 1-7 as the cationic lipid, all four lipid components of the organic phase were dissolved in anhydrous ethanol to a concentration of 10 mg/mL each. The organic phase was prepared according to the molar ratios specified in Table 1, with a total lipid concentration of 4.8 mg/mL. The aqueous phase was prepared by diluting 1 mg/mL Luc mRNA (SEQ ID NO:1) to 70 µg/mL using 25 mM sodium acetate buffer. The two-phase channels of the microfluidic instrument (stainless-steel chip) were thoroughly cleaned with anhydrous ethanol and pure water, followed by rinsing the aqueous phase channel twice with 25 mM sodium acetate buffer. At the start of preparation, approximately 300 µL of the prepared organic phase and aqueous phase solutions were drawn into their respective channels to purge air from the lines. The preparation mixture was then aspirated at a volumetric organic-to-aqueous phase ratio of 1:3, with flow rates set at 4 mL/min for the organic phase and 12 mL/min for the aqueous phase. The first 0.5 mL of the effluent was discarded, and only the subsequent effluent was collected until completion. (The preparation methods of SM-102 LNP and Compound 1 LNP were identical).

**Table 1**

| | Cationic Lipid | PEG Lipid | Cholesterol | DSPC |
|---|---|---|---|---|
| Molar Ratio (%) | 50 | 0.5-5 | 38.5 | 10 |

### 1.1 Ultrafiltration of LNP

The prepared LNP stock solution was diluted with a five-fold volume of PBS. The diluted mixture was subjected to ultrafiltration using an ultrafiltration device by centrifugation at 2000-3000 rpm for 10 minutes, until the retentate volume was concentrated to approximately 1 mL. Subsequently, the ultrafiltration membrane was rinsed with a ten-fold volume of PBS, followed by centrifugation to concentrate the retentate to about 1 mL. This rinsing and concentration step using a ten-fold volume of PBS was repeated once. After this washing procedure, the resulting approximately 1 mL retentate was transferred to a nuclease-free microcentrifuge tube and stored at 4°C for later use.

### 2. Content and Encapsulation Efficiency Determination

### 2.1 Preparation of Standard Curve

The stock mRNA solution was diluted with 1× TE buffer to 2 µg/mL. Various volumes (0, 2, 10, 25, 50, 100 µL) of this standard solution were aliquoted into a black 96-well microplate. Subsequently, the procedure specified in the RNA quantification kit instructions was followed (100 µL of 1× TE and 100 µL of 1× Ribo). Measurements were performed using a microplate reader with excitation and emission wavelengths set at 480 nm and 520 nm, respectively.

### 2.2 Preparation of LNP Measurement Samples

The ultrafiltered LNP was first diluted with 1× TE buffer to an approximate theoretical concentration of 1 µg/mL. For permeabilization, the diluted LNP was treated with Triton X-100 at a final concentration of 1% and incubated at room temperature for 10 minutes. For non-permeabilization control, an equal volume of DEPC water was added to the diluted LNP and incubated at room temperature for 10 minutes. Subsequently, 100 µL aliquots from each sample were transferred to a black 96-well microplate, followed by the addition of 100 µL of 1× Ribo to each well. Each sample was assayed in triplicate. Measurements were performed using a microplate reader with excitation and emission wavelengths set at 480 nm and 520 nm, respectively.

### 3. Particle Size Determination

An appropriate amount of the ultrafiltered LNP was diluted 10-fold with pure water, loaded into a cuvette, and the particle size was measured using a Malvern particle size analyzer.

### 4. Local Expression

Intramuscular Injection: Male C57 mice weighing approximately 20 g each were selected. Prior to injection, hair was removed from the gastrocnemius muscle area of the hind leg. A fine-gauge syringe was then used to administer a dose of 50 µL LNP (containing 10 µg mRNA) into the gastrocnemius muscle. At 4 hours post-injection, 200 µL of luciferase substrate (15 mg/mL) was administered intraperitoneally. The mice were anesthetized with isoflurane and subjected to small animal in vivo imaging for characterization at the 4-hour time point.

Intrasplenic Injection: Male C57 mice weighing approximately 20 g were anesthetized with isoflurane. After being positioned in a supine position, an incision was made in the skin on the right side of the abdomen near the thoracic cavity to expose the spleen. Subsequently, each mouse was administered a dose of 20 µL of Compound 1 LNP (containing 4 µg mRNA) into the spleen using a fine-gauge syringe. At 6 hours post-administration, 200 µL of luciferase substrate (15 mg/mL) was injected intraperitoneally. The mice were then re-anesthetized with isoflurane and subjected to small animal in vivo imaging for characterization. For male SD rats weighing approximately 150 g, each rat received an injection of 30 µL of the formulation (containing 6 µg mRNA), following the same procedure.

Intrakidney Injection: Male C57 mice weighing approximately 20 g were anesthetized with isoflurane. After being placed in a supine position, a skin incision was made on the right side of the abdomen near the thoracic cavity to expose the kidney. Each mouse then received an injection of 20 µL of Compound 1 LNP (containing 4 µg mRNA) directly into the kidney parenchyma using a fine-gauge syringe. At 6 hours post-injection, 200 µL of luciferase substrate solution (15 mg/mL) was administered via intraperitoneal injection. The mice were subsequently re-anesthetized with isoflurane and subjected to small animal in vivo imaging for characterization. For male SD rats weighing approximately 150 g, each animal received an injection of 30 µL of the formulation (containing 6 µg mRNA), following the same procedure.

### Experimental Results

### 1. Particle Size, PDI, and Encapsulation Efficiency (%)

| **LNP** | **Size (d.nm)** | **PDI** | **Encapsulation Efficiency (%)** |
|---|---|---|---|
| Compound 1 | 66.47±1.368 | 0.084±0.045 | 97.78 |
| SM-102 | 70.38±0.468 | 0.055±0.011 | 96.64 |

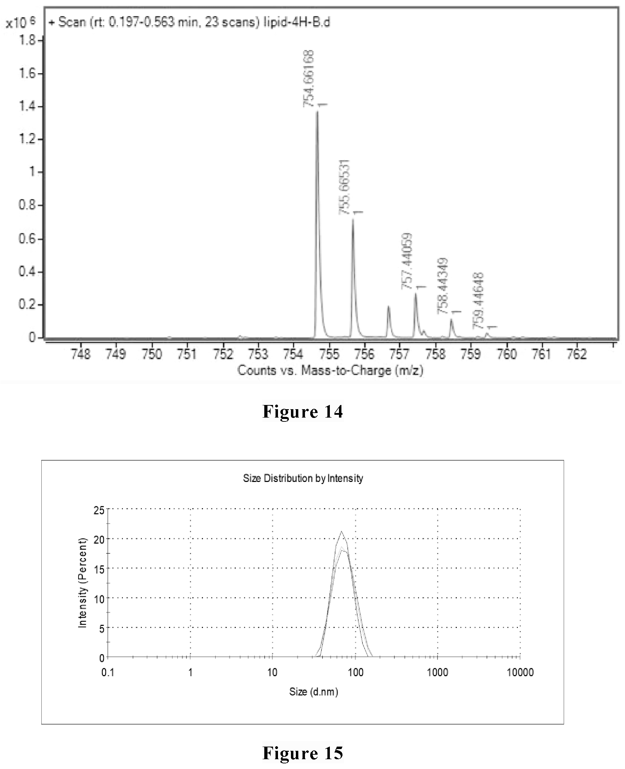

The particle size distribution of the LNP prepared from Compound 1 of the present disclosure is shown in Figure 15.

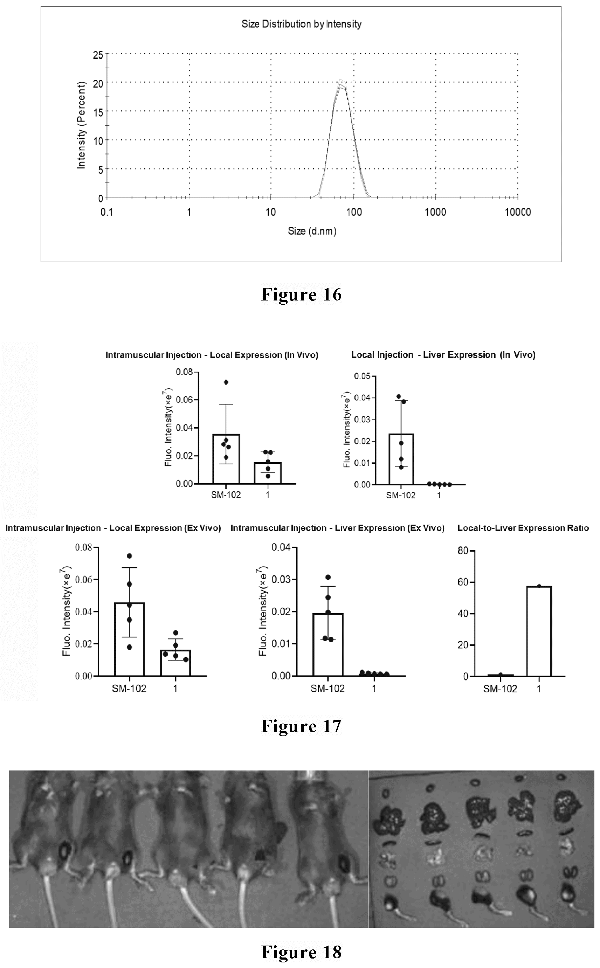

The particle size distribution of SM-102 LNP is shown in Figure 16.

### 2. In Vivo Expression - Intramuscular Injection

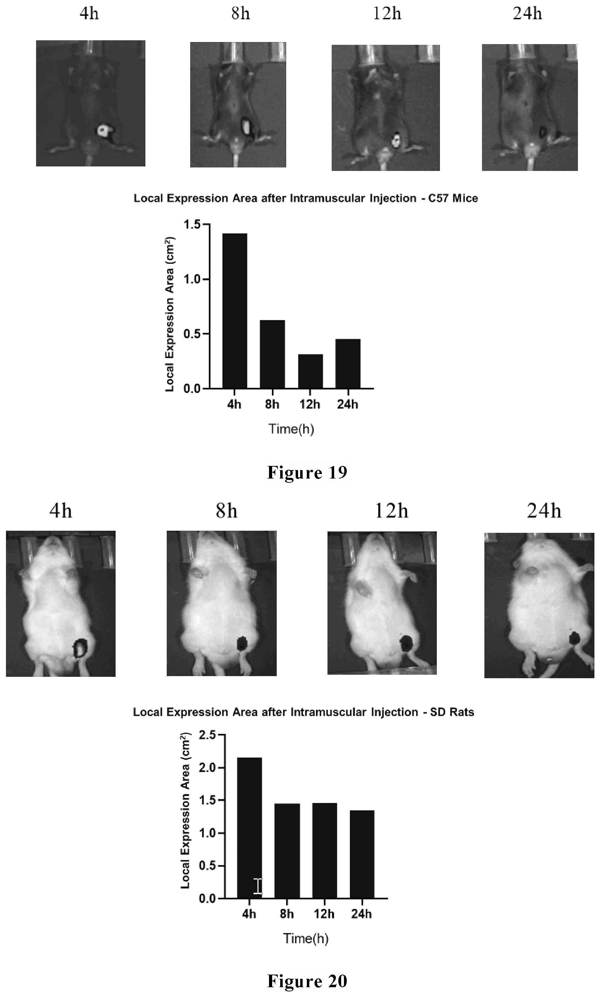

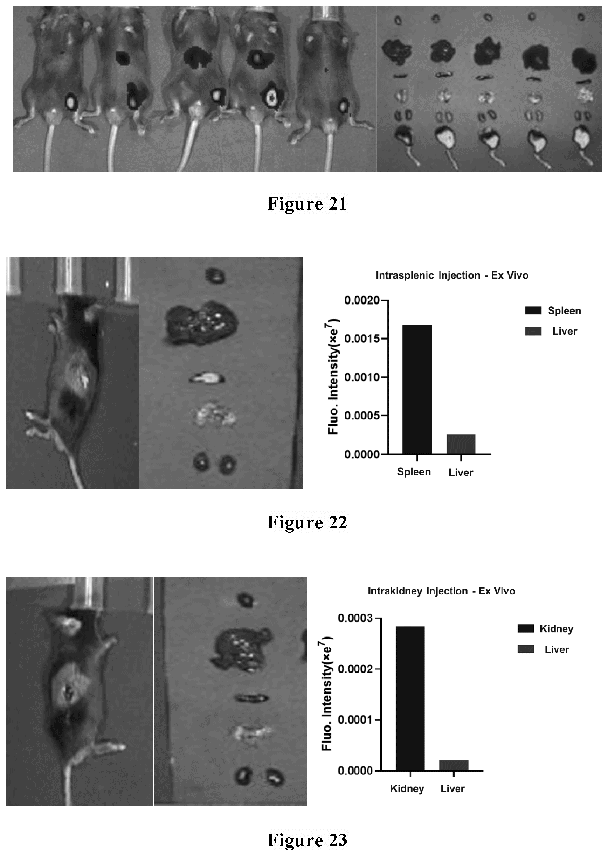

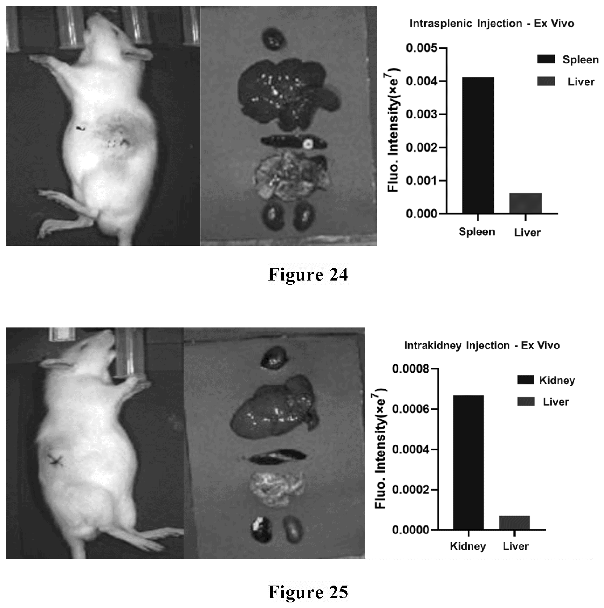

The results are shown in Figures 17-25. It can be observed that after intramuscular injection of Compound 1 LNP, in vivo imaging results in small animals indicate localized expression solely at the injection site, with no detectable expression in other organs.

## Claims

1. A compound of Formula (I): 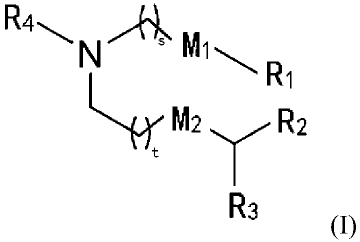 or a salt or isomer thereof, wherein:
s is 5 or 8, t is 7 or 8;
M₁ is -C(O)O- or -C(O)NR-;
M₂ is -C(O)O- or -C(O)NR₅-;
R₁ is C₁₋₁₈ alkyl, C₂₋₁₈ alkenyl, or -CHR₂R₃;
R₂, R₃, and R₅ are each independently selected from: H, C₁₋₁₄ alkyl, and C₂₋₁₄ alkenyl;
R₄ is C₁₋₃ alkyl, -(CH₂)ₙCHR₆R₇, or -CHR₆R₇, wherein R₆ and R₇ are each independently (CH₂)ₙQ, wherein n is 0, 1, 2, 3, or 4, Q is C₁₋₃ alkyl or OR, and R is C₁₋₃ alkyl or H.

2. The compound or a salt or isomer thereof of claim 1, wherein the compound is of Formula (Ia), 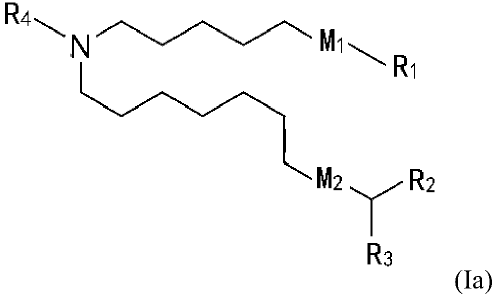 wherein:
s is 5 or 8, t is 7 or 8;
M₁ is -C(O)O- or -C(O)NR-;
M₂ is -C(O)O- or -C(O)NR₅-;
R₁ is C₁₋₁₈ alkyl, C₂₋₁₈ alkenyl, or -CHR₂R₃;
R₂, R₃, and R₅ are each independently selected from: H, C₁₋₁₄ alkyl, and C₂₋₁₄ alkenyl;
R₄ is C₁₋₃ alkyl, -(CH₂)ₙCHR₆R₇, or -CHR₆R₇, wherein R₆ and R₇ are each independently (CH₂)ₙQ, wherein n is 0, 1, 2, 3, or 4, Q is C₁₋₃ alkyl or OR, and R is C₁₋₃ alkyl or H.

3. The compound or a salt or isomer thereof of claim 1 or 2, wherein:
M₁ is -C(O)O- or -C(O)NH-; and/or
M₂ is -C(O)NR₅-, R₂ is H, R₃ is C₆ alkyl, R₅ is C₇ alkyl; and/or
R₁ is C₁₋₁₄ alkyl; and/or
Q is OH; and/or
R₂ and R₃ are each independently selected from C₁₋₁₂ alkyl.

4. A nanoparticle composition comprising a lipid component, wherein the lipid component comprises the compound or a salt or isomer thereof of any one of claims 1 to 3,
preferably, wherein the lipid component further comprises a phospholipid, a PEG lipid, and a structural lipid.

5. The nanoparticle composition of claim 4, wherein the nanoparticle composition further comprises a therapeutic and/or prophylactic agent.

6. A pharmaceutical composition comprising the nanoparticle composition of claim 4 or 5 and a pharmaceutically acceptable excipient.

7. A non-therapeutic method for producing a polypeptide in a cell, the method comprising contacting the cell with a nanoparticle composition, wherein the nanoparticle composition comprises: (1) a lipid component comprising a phospholipid, a PEG lipid, a structural lipid, and the compound or a salt or isomer thereof of any one of claims 1 to 3, and (2) an mRNA encoding the polypeptide, whereby the mRNA is capable of being translated in the cell to produce the polypeptide,
preferably, wherein the cell is a mammalian cell.

8. A method of preparing the compound or a salt or isomer thereof of any one of claims 1 to 3 or a method of preparing the nanoparticle composition of claim 4 or 5.

9. Use of the compound or a salt or isomer thereof of any one of claims 1 to 3 and/or the nanoparticle composition of claim 4 or 5 in the manufacture of a medicament for treating or preventing a disease or disorder,
preferably, wherein the medicament is a locally acting medicament,
preferably, wherein the nanoparticle composition, or the nanoparticle composition formed from the compound of Formula (I), is for delivering the therapeutic and/or prophylactic agent to a local region,
preferably, wherein the disease or disorder benefits from the delivery of a therapeutic and/or prophylactic agent to a local region.

10. The use according to claim 9, wherein the local region is an organ or tissue,
preferably, wherein the local region is a liver, spleen, kidney, lung, muscle, or bone.
